(19) Europäisches Patentamt
European Patent Office
Office européen des brevets

(11) Publication number: **0 029 306**
**B1**

(12) **EUROPEAN PATENT SPECIFICATION**

(45) Date of publication of patent specification: **25.07.84**

(21) Application number: **80303753.0**

(22) Date of filing: **23.10.80**

(51) Int. Cl.$^3$: **C 07 C 87/22,**
C 07 D 249/14,
C 07 D 295/08,
C 07 D 405/12,
C 07 D 409/12,
A 61 K 31/04,
A 61 K 31/41,
A 61 K 31/445
//(C07D405/12, 307/52,
249/14), (C07D409/12,
333/20, 249/14)

(54) **Aminoalkyl compounds, their production and pharmaceutical compositions containing them.**

(30) Priority: **23.10.79 GB 7936767**
**05.06.80 GB 8018403**
**27.08.80 GB 8027742**

(43) Date of publication of application:
**27.05.81 Bulletin 81/21**

(45) Publication of the grant of the patent:
**25.07.84 Bulletin 84/30**

(84) Designated Contracting States:
**BE CH DE FR IT LI NL**

(56) References cited:
**AT - B - 342 587**
**US - A - 3 686 201**
**US - A - 3 813 400**

(73) Proprietor: **GLAXO GROUP LIMITED**
**Clarges House 6-12 Clarges Street**
**London W1Y 8DH (GB)**

(72) Inventor: **Bradshaw, John**
**22 Whiteley Close, Dane End**
**Ware Hertfordshire (GB)**
Inventor: **Clitherow, John Watson**
**54 Gilders**
**Sawbridgeworth Hertfordshire (GB)**
Inventor: **MacFarlane MacKinnon, John Wilson**
**31 Trapstyle Road**
**Ware Herts (GB)**
Inventor: **Hayes, Roger**
**5 Sandringham Road**
**Potters Bar Herts (GB)**
Inventor: **Carey, Linda**
**15 Roan Walk**
**Royston Hertfordshire (GB)**

(74) Representative: **Marchant, James Ian et al,**
**Elkington and Fife High Holborn House 52/54**
**High Holborn**
**London WC1V 6SH (GB)**

**EP 0 029 306 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to novel heterocyclic derivatives having action on histamine receptors, to processes for the preparation thereof, to pharmaceutical compositions containing them and to thier use in therapeutics.

Certain novel heterocyclic derivatives have now been found which have potent activity as $H_2$-antagonists. These compounds, which are more particularly described below, for example show inhibition of the secretion of gastric acid when this is stimulated via histamine receptors (Ash and Schild, Brit. J. Pharmacol. Chemother, 1966, *27*, 427). Their ability to do so can be demonstrated in the perfused rat stomach using the method described in German Offenlegungsschrift No. 2,734,070, modified by the use of sodium pentobarbitone (50 mg/kg) as anaesthetic instead of urethane, and in conscious dogs equipped with Heidenhain pouches using the method described by Black *et al*, Nature 1972 *236*, 385. Furthermore the compounds antagonise the effect of histamine on the contraction frequency of isolated guinea pig right atrium but do not modify histamine induced contractions of isolated gastrointestinal smooth muscle which are modiated via $H_1$-receptors.

Compounds with histamine $H_2$-blocking activity may be used in the treatment of conditions where there is an advantage in lowering gastric acidity, particularly in gastric and peptic ulceration, as a prophylactic measure in surgical procedures, and in the treatment of allergic and inflammatory conditions where histamine is a known mediator. Thus they may be used for example, either alone, or in combination with other active ingredients in the treatment of allergic and inflammatory conditions of the skin.

The present invention provides compounds of the general formula (I)

$$R_1R_2N—Alk—Q—X\ (CH_2)_n\ Y\ (CH_2)_m\ NH—B \qquad (I)$$

and physiologically acceptable salts and hydrates thereof in which

B represents either the group

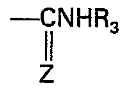 or the group 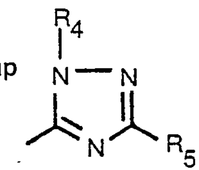 ;

$R_1$ represents $C_{3-8}$ cycloalkyl; aryl $C_{1-6}$ alkyl; trifluoro $C_{1-6}$ alkyl; heteroarylalkyl wherein the heteroaryl part is a monocyclic, unsaturated ring containing 5 or 6 atoms selected from carbon, oxygen, nitrogen and sulphur linked to the alkylene chain either through carbon or nitrogen and optionally substituted by $C_{1-16}$ straight or branched saturated or alkylene chain optionally substituted by a hydroxy, amino, $C_{1-6}$ alkylamino, or di $C_{1-6}$ alkylamino group; or $R_1$ represents a $C_{1-16}$ straight or branched saturated alkylene chain interrupted by an oxygen atom; with the proviso that when the alkylene chain is interrupted by an oxygen atom, then there must be at least two carbon atoms between that group and the nitrogen atom to which the group $R_1$ is attached;

$R_2$ represents hydrogen or a $C_{1-4}$ alkyl group;

or $R_1$ and $R_2$ together with the nitrogen atom to which they are attached form a 5—10 membered ring which may be saturated or may contain at least one double bond, may be unsubstituted or may be substituted by one or more $C_{1-3}$ alkyl groups e.g. methyl or a hydroxy group and/or may contain another heteroatom selected from oxygen and sulphur;

Alk represents a straight or branched alkylene chain of 1 to 6 carbon atoms, preferably 1 to 4 carbon atoms;

Q represents a furan or thiophen ring in which incorporation into the rest of the molecule is through bonds at the 2- and 5- positions, the furan ring optionally bearing a further substituent $R_7$ adjacent to the group $R_1R_2$—N—Alk-, or Q represents a benzene ring in which incorporation into the rest of the molecule is through bonds at the 1- and 3- or 1- and 4- positions;

$R_7$ represents halogen, or $C_{1-4}$ alkyl which may be substituted by hydroxy or $C_{1-4}$ alkoxy;

X and Y, which may be the same or different, each represent oxygen or sulphur, or one of X and Y represents —$CH_2$— and the other represents oxygen or sulphur;

n and m, which may be the same or different, each represent 2 or 3;

$R_3$ represents hydrogen, $C_{1-6}$ alkyl, $C_{3-6}$ alkenyl, or $C_{1-6}$ alkoxy $C_{1-6}$ alkyl;

Z represents $CHNO_2$ or $NR_8$ where $R_8$ is nitro, cyano, $C_{1-6}$ alkylsulphonyl or arylsulphonyl;

$R_4$ represents hydrogen, $C_{1-6}$ alkyl, $C_{3-6}$ alkenyl, aryl $C_{1-6}$ alkyl, or $C_{2-6}$ alkyl substituted by hydroxy or $C_{1-6}$ alkoxy; and

$R_5$ represents hydrogen, $C_{1-6}$ alkyl, $C_{3-6}$ alkenyl, aryl $C_{1-6}$ alkyl, hydroxy $C_{1-6}$ alkyl, acyloxy $C_{1-6}$ alkyl (where the acyl portion is aroyl, aryl $C_{2-7}$ alkanoyl or $C_{1-6}$ alkanoyl), $C_{1-6}$ alkoxy $C_{1-6}$ alkyl, aryloxy, $C_{1-6}$ alkyl, aryl $C_{1-6}$ alkyloxy $C_{1-6}$ alkyl, amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy or the group $NR_9R_{10}$ where $R_9$ represents hydrogen or $C_{1-6}$ alkyl;

$R_{10}$ represents hydrogen, $C_{1-6}$ alkyl, $C_{1-6}$ alkyl substituted by hydroxy or $C_{1-3}$ alkoxy, $C_{3-6}$ alkenyl, aryl $C_{1-6}$ alkyl or heteroaryl $C_{1-6}$ alkyl, or $R_{10}$ may represent the group $COR_{11}$ where $R_{11}$ represents hydrogen, $C_{1-6}$ alkyl, aryl, aryl $C_{1-6}$ alkyl, $C_{1-6}$ alkoxy, heteroaryl or heteroaryl $C_{1-6}$ alkyl or $R_{10}$ represents the group $SO_2R_{12}$ where $R_{12}$ represents $C_{1-6}$ alkyl or aryl, or $R_{10}$ represents the group

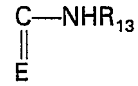

where E is oxygen or sulphur and

$R_{13}$ represents hydrogen, $C_{1-6}$ alkyl, $C_{3-8}$ cycloalkyl, aryl or aryl $C_{1-6}$ alkyl,

or $R_9$ and $R_{10}$ taken together represent the group $=CR_{14}R_{15}$ where $R_{14}$ represents aryl or heteroaryl and $R_{15}$ represents hydrogen or alkyl; and wherein

aryl as a group or part of a group means phenyl or phenyl substituted with one or more $C_{1-3}$ alkyl or $C_{1-3}$ alkoxy groups or halogen atoms;

heteroaryl as a group or part of a group within the definition of the group $R_5$ means a 5 or 6 membered monocyclic unsaturated ring which may contain one or more heteroatoms selected from oxygen, nitrogen or sulphur.

The term "alkyl" as a group or part of a group means that the group is straight or branched and has unless otherwise stated preferably 1 to 4 carbon atoms, e.g. methyl or ethyl. Examples of acyloxyalkyl groups include acetoxymethyl, formyloxymethyl, benzoloxymethyl and phenylacetoxymethyl. The term "heteroaryl" as a group or part of a group when applied to the group $R_5$ can be for example, furyl, pyridyl, thiazolyl or thienyl.

The invention includes the compounds of formula (I) in the form of physiologically acceptable salts with inorganic and organic acids. Particularly useful salts include hydrochlorides, hydrobromides and sulphates, methanesulphonates, acetates, maleates, succinates, tartrates, benzoates, citrates and fumarates. The compounds of formula (I) and their salts may also form hydrates, which hydrates are also to be considered as part of the invention. The compounds of formula (I) can exhibit tautomerism and the formula is intended to cover all tautomers. Where optical isomers may exist the formula is intended to cover all diastereoisomers and optical enantiomers.

The compounds according to the invention, preferably in the form of a salt, may be formulated for administration in any convenient way and the invention includes within its scope pharmaceutical compositions containing at least one compound according to the invention adapted for use in human or veterinary medicine. Such compositions may be formulated in a conventional manner using one or more pharmaceutically acceptable carriers or excipients. Such compositions may also contain if required other active ingredients, e.g. $H_1$-antagonists.

Thus the compounds according to the invention may be formulated for oral, buccal, topical, parenteral or rectal administration. Oral administration is preferred.

For oral administration, the pharmaceutical composition may take the form of for example, tablets, capsules, powders, solutions, syrups or suspensions prepared by conventional means with acceptable excipients. For buccal administration the composition may take the form of tablets or lozenges formulated in conventional manner.

The compounds of the invention may be formulated for parenteral administration by bolus injection or continuous infusion. Formulations for injection may be presented in unit dosage form in ampoules, or in multi-dose containers, with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for reconstitution with a suitable vehicle, e.g. sterile pyrogen-free water before use.

The compounds of the invention may also be formulated in rectal compositions such as suppositories or retention enemas, e.g. containing conventional suppository bases such as cocoa butter or other glyceride.

For topical application, the compounds of the invention may be formulated as ointments, creams, gels, lotions, powders or sprays in a conventional manner.

For internal administration a convenient daily dosage regime of the compounds according to the invention would be 1 to 4 doses of the total of some 5 mg to 2 g per day, preferably 5 to 500 mg per day dependent upon the condition of the patient.

Examples of suitable meanings for the groups $R_1$, $R_2$ and $R_7$ are as follows:

$R_1$: alkyl containing up to 16 carbon atoms (e.g. methyl, ethyl, propyl, butyl, pentyl, hexyl, heptyl or decyl), $C_{5-7}$ cycloalkyl (e.g. cyclopentyl, cyclohexyl or cycloheptyl), $C_{3-6}$ alkenyl (e.g. allyl or 3,3-dimethylallyl), aryl $C_{1-6}$ alkyl (e.g. phenylalkyl such as benzyl or phenethyl), $C_{1-4}$ alkyl subsituted by a trifluoromethyl group (e.g. 2,2,2-trifluoroethyl), hydroxy $C_{2-4}$ alkyl (e.g. 3-hydroxypropyl), $C_{1-3}$ alkoxy $C_{2-4}$ alkyl (e.g. methoxyethyl or ethoxyethyl), or di-$C_{1-3}$ alkylamino $C_{1-6}$ alkyl (e.g. dimethylaminoethyl, diethylaminoethyl or dimethylaminopropyl), or heteroarylalkyl where the heterocyclic portion represents a furyl, thienyl, pyrrolyl, pyridinyl, pyrimidinyl, triazinyl, oxazolyl, triazolyl or thiazolyl, ring and the alkylene portion is methylene, ethylene or propylene;

$R_2$: hydrogen or $C_{1-4}$ alkyl (e.g. methyl or ethyl); or

$R_1R_2N$ may represent a 5—8 membered ring optionally containing one double bond and/or substituted by one or two $C_{1-3}$ alkyl (e.g. methyl) groups or a hydroxy group and/or containing an oxygen or sulphur atom (e.g. pyrrolidino, piperidino, hexamethylenimino, heptamethylenimino, tetrahydropyridino, 4-hydroxypiperidino, 4-$C_{1-3}$ alkylpiperidino (e.g. 4-methylpiperidino), morpholino, 2,6-di-$C_{1-3}$ alkylmorpholino (e.g. 2,6-dimethylmorpholino) or thiomorpholino);

$R_7$: bromine atom or a $C_{1-3}$ alkyl group (e.g. methyl, ethyl or isopropyl) or a $C_{1-3}$ alkoxy $C_{1-3}$ alkyl group (e.g. methoxymethyl).

When B represents the group

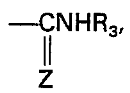

examples of suitable meanings for $R_3$ and Z are as follows:

$R_3$ hydrogen, $C_{1-4}$ alkyl (e.g. methyl or ethyl) or $C_{1-3}$ alkoxy $C_{2-4}$ alkyl (e.g. methoxyethyl)

Z: $CHNO_2$ or $NR_8$ where $R_8$ is nitro, cyano or $C_{1-4}$ alkylsulphonyl (e.g. methylsulphonyl).

When B represents the group

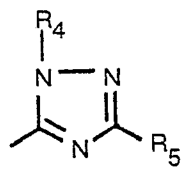 ;

examples of suitable meanings for $R_4$ and $R_5$ are as follows:

$R_4$: hydrogen, $C_{1-4}$ alkyl (e.g. methyl, ethyl or propyl) or hydroxy $C_{2-4}$ alkyl (e.g. 2-hydroxyethyl);

$R_5$: hydrogen, hydroxy, $C_{1-4}$ alkyl (e.g. methyl, ethyl or isobutyl), hydroxy $C_{1-4}$ alkyl (e.g. hydroxymethyl, 2-hydroxyethyl or 1-hydroxy-1-methylethyl), $C_{1-3}$ alkoxy $C_{1-4}$ alkyl (e.g. methoxymethyl or methoxyethyl), phenyl $C_{1-3}$ alkyl (e.g. benzyl or phenethyl), $C_{2-4}$ alkanoyloxy $C_{1-4}$ alkyl (e.g. acetoxymethyl), amino $C_{1-4}$ alkyl (e.g. aminomethyl), amino, $C_{1-4}$ alkylamino (e.g. methylamino or ethylamino) or di-$C_{1-4}$ alkylamino (e.g. dimethylamino, diethylamino or dipropylamino), phenyl $C_{1-3}$ alkylamino (e.g. benzylamino), or a heteroaryl $C_{1-3}$ alkylamino group where the heteroaryl ring is 5 or 6 membered and contains one heteroatom (e.g. 3- or 4- pyridylmethyl); or the group $NHCOR_{11}$ where $R_{11}$ represents hydrogen, $C_{1-3}$ alkyl (e.g. methyl or ethyl), $C_{1-3}$ alkoxy (e.g. methoxy or ethoxy), furyl, pyridyl, thiazolyl, thienyl, or phenyl optionally substituted by a $C_{1-3}$ alkyl (e.g. methyl) or $C_{1-3}$ alkoxy (e.g. methoxy) group; or the group $NHSO_2R_{12}$ where $R_{12}$ represents $C_{1-3}$ alkyl (e.g. methyl), or phenyl optionally substituted by a $C_{1-3}$ alkyl (e.g. methyl) or $C_{1-3}$ alkoxy (e.g. methoxy) group; or the group $—NHCONHR_{13}$ where $R_{13}$ is $C_{1-3}$ alkyl (e.g. methyl), $C_{5-7}$ cycloalkyl (e.g. cyclohexyl), or phenyl optionally substituted by a $C_{1-3}$ alkyl (e.g. methyl) or $C_{1-3}$ alkoxy (e.g. methoxy) group, or the group $N{=}CHR_{14}$ where $R_{14}$ is a phenyl or pyridyl (e.g. 3- or 4-pyridyl) group.

The group Alk may be for example the group $(CH_2)_p$ where p is 1, 2 or 3.

In particular the groups $R_1$ and $R_2$ may have meanings as follows:

$R_1$: $C_{1-7}$ alkyl (e.g. methyl, propyl, butyl, isobutyl, or heptyl), $C_{1-4}$ alkyl substituted by a trifluoromethyl group (e.g. 2,2,2-trifluoroethyl), $C_{2-4}$ alkyl substituted by hydroxy or a di $C_{1-3}$ alkylamino group (e.g. 3-hydroxypropyl or dimethylaminoethyl), $C_{5-7}$ cycloalkyl (e.g. cyclohexyl), $C_{3-6}$ alkenyl (e.g. allyl), phenyl $C_{1-3}$ alkyl (e.g. benzyl), or a heteroaryl $C_{1-3}$ alkyl group where the heteroaryl ring is 5 or 6 membered and contains one heteroatom (e.g. 2-furylmethyl);

$R_2$: hydrogen or methyl; or

$R_1R_2N$ may represent a 5 to 7 membered ring optionally containing a double bond or an alkyl (e.g. methyl substituent (e.g. piperidino, 4-methylpiperidino pyrrolidino, hexamethylenimino or tetrahydropyridino.

When B represents the group

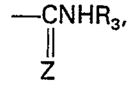

$R_3$ and Z may, in particular, have meanings as follows:

$R_3$: hydrogen or $C_{1-3}$ alkyl (e.g. methyl)

Z: $CHNO_2$ or $NR_8$ where $R_8$ is cyano, nitro or methylsulphonyl.

When B represents the group

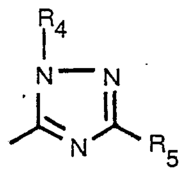 ;

$R_4$ and $R_5$ may, in particular, have meanings as follows:

$R_4$: hydrogen, methyl, ethyl or 2-hydroxyethyl;

$R_5$: hydroxy, phenyl $C_{1-3}$ alkyl (e.g. benzyl), $C_{1-4}$ alkyl substituted by hydroxy, $C_{1-3}$ alkoxy, $C_{2-4}$ alkanoyloxy or amino (e.g. hydroxymethyl, 2-hydroxyethyl, acetoxymethyl or aminomethyl); amino, di-$C_{1-4}$ alkylamino (e.g. diethylamino), $NHCOR_{11}$ where $R_{11}$ represents hydrogen, $C_{1-3}$ alkyl, (e.g. methyl), $C_{1-3}$ alkoxy (e.g. ethoxy) or phenyl; —$NHCONHR_{13}$ where $R_{13}$ represents phenyl; or $N{=}CHR_{14}$ where $R_{14}$ is phenyl or pyridyl (e.g. 4-pyridyl). Alk is particularly a methylene or ethylene group, more particularly methylene.

The group Q is preferably a benzene ring in which incorporation into the rest of the molecule is through bonds at the 1- and 3-position.

One of X and Y is preferably oxygen and the other is preferably oxygen or methylene.

n and m are preferably both two.

When B represents the group

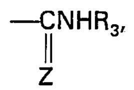

Z is preferably $CHNO_2$ and $R_3$ is more particularly alkyl (e.g. methyl).

A preferred group of compounds of formula (I) are triazoles of formula (II)

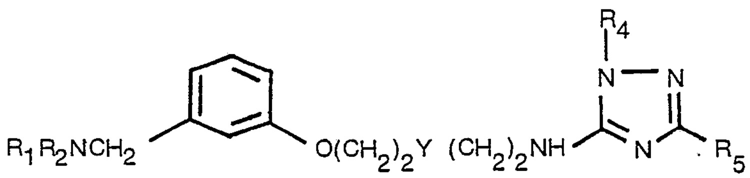 (II)

where $R_1$ and $R_2$ are methyl groups or together with the nitrogen atom to which they are attached form a pyrrolidino, piperidino, hexamethylenimino, tetrahydropyridino or 4-methylpiperidino group; $R_4$ is hydrogen or methyl; and $R_5$ is an amino, hydroxy $C_{1-6}$ alkyl, $C_{2-7}$ alkanoylamino, $C_{2-7}$ alkanoyloxy $C_{1-6}$ alkyl, $C_{1-6}$ alkoxy $C_{1-6}$ alkyl, amino $C_{1-6}$ alkyl, benzyl, formamido, $C_{1-6}$ alkoxycarbonylamino, hydroxy, aroylamino or phenylcarbamoylamino group; and Y represents oxygen or methylene.

A further preferred group of compounds of formula (I) are those of formula (III)

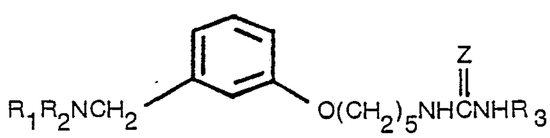 (III)

in which $R_1R_2N$ forms a pyrrolidino, piperidino, hexamethylenimino, tetrahydropyridino or 4-methylpiperidino group; Z represents $CHNO_2$ or $NR_8$ where $R_8$ is methylsulphonyl, more preferably $CHNO_2$; and $R_3$ represents hydrogen, $C_{1-4}$ alkyl or $C_{1-3}$ alkoxy $C_{2-4}$ alkyl, more preferably $C_{1-4}$ alkyl e.g. methyl.

A particularly preferred compound is N-[5-[3-(-piperidinylmethyl)phenoxy]pentyl]-N'-methyl-2-nitro-1,1-ethenediamine and its physiologically acceptable salts.

According to one aspect the invention provides compounds of formula (I) in which B represents

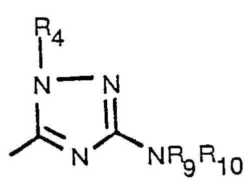

$R_1$ represents $C_{3-8}$ cycloalkyl, aryl $C_{1-6}$ alkyl, $C_{3-6}$ alkenyl, trifluoro $C_{1-6}$ alkyl, heteroaryl $C_{1-4}$ alkyl or $C_{1-6}$ alkyl substituted by $C_{3-8}$ cycloalkyl, amino, $C_{1-6}$ alkylamino or di $C_{1-6}$ alkylamino; or $R_1$ represents a $C_{1-16}$ straight or branched saturated alkylene chain which may be optionally substituted by a hydroxy group; or $R_1$ represents a $C_{1-16}$ straight or branched saturated alkylene chain interrupted by

an oxygen atom, with the proviso that when the alkylene chain is interrupted by an oxygen atom, then there must be at least two carbon atoms between that group and the nitrogen atom to which the group $R_1$ is attached.

$R_2$ represents hydrogen or a $C_{1-4}$ alkyl group;

or $R_1$ and $R_2$ together with the nitrogen atom to which they are attached form a 5—10 membered ring which may be saturated or may contain at least one double bond, may be unsubstituted or may be substituted by one or more $C_{1-3}$ alkyl groups or a hydroxy group and/or may contain another heteroatom selected from oxgyen and sulphur; and

$R_9$ represents hydrogen or $C_{1-6}$ alkyl;

$R_{10}$ represents hydrogen, $C_{1-6}$ alkyl, $C_{1-6}$ alkyl substituted by hydroxy or $C_{1-3}$ alkoxy, $C_{3-6}$ alkenyl, aryl $C_{1-6}$ alkyl or heteroaryl $C_{1-6}$ alkyl;

or $R_9$ and $R_{10}$ together represent the group $=CR_{14}R_{15}$ where $R_{14}$ represents aryl or heteroaryl and $R_{15}$ represents hydrogen or $C_{1-6}$ alkyl;

wherein aryl and heteroaryl (within the definitions of $R_1$ and $R_5$) are as defined above in connection with formula (I).

According to another aspect the invention provides compounds of formula (I) in which B represents

$$-\underset{\underset{Z}{\|}}{C}NHR_3;$$

$R_1$ represents $C_{3-8}$ cycloalkyl; aryl $C_{1-6}$ alkyl; trifluoro $C_{1-6}$ alkyl; heteroaryl $C_{1-4}$ alkyl (as defined above in connection with formula (I)) or $C_{1-6}$ alkyl substituted by $C_{3-8}$ cycloalkyl, amino, $C_{1-6}$ alkylamino or di $C_{1-6}$ alkylamino;

or $R_1$ represents a $C_{1-16}$ straight or branched saturated alkylene chain optionally substituted by a hydroxy group; or $R_1$ represents a $C_{1-16}$ straight or branched saturated alkylene chain interrupted by an oxygen atom; with the proviso that when the alkylene chain is interrupted by an oxygen atom, then there must be at least two carbon atoms between that group and the nitrogen atom to which the group $R_1$ is attached;

$R_2$ represents hydrogen or a $C_{1-4}$ alkyl group;

or $R_1$ and $R_2$ together with the nitrogen atom to which they are attached form a 5—10 membered ring which may be saturated or may contain at least one double bond, may be unsubstituted or may be substituted by one or more $C_{1-3}$ alkyl groups or a hydroxy group and/or may contain another heteroatom selected from oxgen and sulphur.

It will be appreciated in the methods for the preparation of the compounds of the formula (I) given below that for certain reaction steps it may be necessary to protect various reactive substituents in the starting materials for a particular reaction and subsequently to remove the protecting group. Such protection and subsequent deprotection may be particularly pertinent when $R_2$ and/or $R_3$ are hydrogen, and/or when $R_4$ is an alkyl group bearing a hydroxy substituent and/or $R_5$ is an alkyl group bearing a hydroxy or a primary or secondary amino substituent. Standard protection and deprotection procedures can be employed.

For example formation of phthalimide (in the case of primary amines), benzyl, benzyloxycarbonyl, or trichloroethoxycarbonyl derivatives. Subsequent cleavage of the protecting group is achieved by conventional procedures. Thus a phthalimide group may be cleaved by treatment with a hydrazine e.g. hydrazine hydrate or a primary amine for example methylamine, benzyl or benzyloxycarbonyl derivatives may be cleaved by hydrogenolysis in the presence of a catalyst, e.g. palladium, and trichloroethoxycarbonyl derivatives may be cleaved by treatment with zinc dust.

In describing the processes which may be used for preparing the compounds of formula (I) or intermediates useful in the preparation thereof, any of $R_1$ to $R_{15}$, Alk, Q, X, Y, Z, E, n and m are as defined in formula (I) unless otherwise stated.

Compounds of formula (I) where B represents the group

$$-\underset{\underset{Z}{\|}}{C}NHR_3$$

may be made by reacting an amine of the formula $R_{16}NH_2$ (IV) with a compound of general formula (V)

$$R_{17}NH\underset{\underset{Z}{\|}}{C}-P \qquad (V)$$

wherein one of the groups $R_{16}$ and $R_{17}$ represents the group $R_1R_2NAlkQX(CH_2)_nY(CH_2)m$- and the other

represents the group $R_3$, and P is a leaving group such as halogen, thioalkyl (preferably thiomethyl) or alkoxy.

Compounds of formula (V) may be prepared by reacting the amine (IV) with a compound of formula (VI).

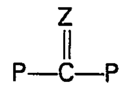

(VI)

where P is as defined in formula (V).

The above reactions may be effected in the absence or presence of a solvent e.g. ethanol, dioxan, acetonitrile, water or aqueous ethanol at a temperature from ambient to reflux. In the absence of a solvent the reaction may be carried out by heating a mixture of the reactants at 100—120°C.

Compounds of formula (I) in which B represents the group

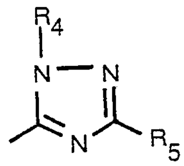

;

where $R_5$ is other than an alkoxy or acyloxyalkyl group or the group $—N=CR_{14}R_{15}$ may be prepared by cyclisation of a compound of formula (VII)

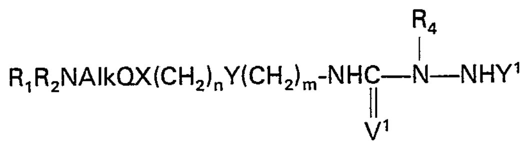

(VII)

in which $V^1$ is

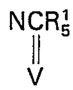

or NCN and $Y^1$ is hydrogen, where V is oxygen or sulphur and $R_5^1$ is a group as defined in formula (I) for $R_5$ or a group convertible thereto under the conditions of the cyclisation reaction or represents halogen or alkoxy; or $V^1$ is NH and $Y^1$ is

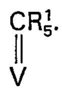

In a particular embodiment of the above process compounds of formula (I) in which the group B represents

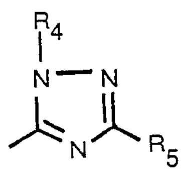

;

where $R_5$ is amino, hydroxy or a carbon-linked group may conveniently be prepared by cyclisation of a compound of formula

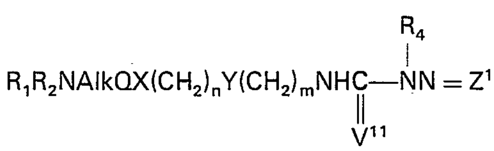

(VIII)

where $V^{11}$ represents the group NCN or

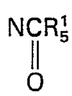

and $Z^1$ represents two hydrogen atoms. The reaction may be carried out in the absence or presence of a suitable solvent, for example, toluene, ethanol, methanol, isopropanol, acetonitrile or water, at a temperature from room temperature to reflux.

It may be convenient to prepare *in situ* compounds of formula (VIII) in which $Z^1$ represents two hydrogen atoms by treating a compound of formula (VIII) where $Z^1$ represents a divalent protecting group which can readily be removed to yield two hydrogen atoms, for example a benzylidene group, with an acid, e.g. hydrochloric acid, preferably with heating and under such conditions cyclisation to give compounds of formula (I) will normally occur.

When preparing compounds of formula (I) in which $R_5$ is a hydroxyalkyl group it is preferable that in the intermediate (VIII) the hydroxy group is in protected form e.g. as an acyloxy derivative such as alkanoyloxy or aroyloxy derivative. The protecting group will normally be removed during the cyclisation process.

Compounds of formula (I) where Alk represents $CH_2$ and where B represents the group

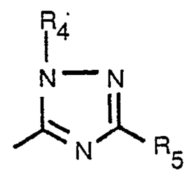 ;

in which $R_5$ is other than the group $-N=CR_{14}R_{15}$ may be prepared by reducing the intermediate formed from reacting a compound of formula (IX)

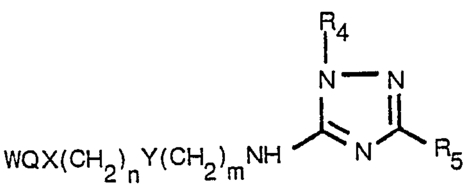

in which W represents the group —CHO with a reagent $R_1R_2NH$. Reaction may be effected by treatment with ammonia or an amine $R_1R_2NH$ in a solvent e.g. tetrahydrofuran or an alkanol such as ethanol or methanol, followed by reduction e.g. with a hydride reducing agent such as an alkali metal or alkaline earth metal borohydride, e.g. sodium borohydride, or aluminium hydride or lithium aluminium hydride or with hydrogen and a metal catalyst e.g. palladium or platinum.

Alternatively, for the production of compounds of formula (I) in which $R_5$ is other than $-N=CR_{14}R_{15}$, a compound of formula (I) in which $R_1$ is hydrogen may be reacted with an aldehyde $R_1^1$—CHO under the reducing conditions described above; the group $R_1^1$ having a meaning such that the resultant group $R_1^1CH_2$ represents a group $R_1$ as defined above. This method applies particularly to the preparation of compounds of formula (I) in which $R_1$ is heteroaralkyl or an optionally substituted alkyl chain containing up to 16 carbon atoms.

In certain instances compounds of formula (I) where B represents the group

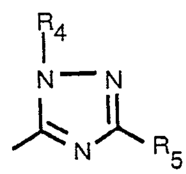 ;

may be prepared by methods involving interconversion of the substituent $R_5$.

Thus compounds in which $R_5$ is an acyloxyalkyl group may be prepared by treating the corresponding hydroxyalkyl compound with an appropriate acid, e.g. acetic or benzoic acid at elevated temperatures e.g. 80—120°C in the absence or presence of a solvent such as toluene.

Compounds of formula (I) in which $R_5$ represents the group $-N=CR_{14}R_{15}$ may be prepared from compounds of formula (I) in which $R_5$ is a $NH_2$ group by reaction with an aldehyde or ketone $R_{14}R_{15}CO$ in a solvent such as benzene, ethanol, or methanol. The reaction is preferably carried out with heating, e.g. at reflux.

Compounds of formula (I) in which $R_5$ is the group $NR_9R_{10}$ where $R_{10}$ is $-COR_{11}$, $-SO_2R_{12}$ or $-C(=E)NHR_{13}$ may be prepared by treating a compound of formula (I) in which $R_5$ is the group $-NHR_9$ and $R_1$, $R_2$, $R_4$ and $R_9$ are as defined in formula (I) or are groups readily convertible thereto with a reagent capable of replacing the hydrogen atom in the group $NHR_9$ by the group $R_{10}$ where $R_{10}$ is as defined above.

Thus for example reaction with an activated derivative of either a carboxylic acid $R_{11}COOH$ or a sulphonic acid $R_{12}SO_3H$, or reaction with an isocyanate or isothiocyanate $R_{13}NCE$ in which $R^1_{13}$ has any of the meanings defined for $R_{13}$ in formula (I) except hydrogen or represents an alkali metal atom such as potassium or sodium or an alkoxycarbonyl group, e.g. ethoxycarbohyl, gives a compound of formula (I) in which $R_{10}$ is respectively the group $COR_{11}$, $SO_2R_{12}$ or $C(=E)NHR_{13}$.

Suitable activated derivatives include acid halides e.g. acid chlorides, alkylchloroformates, acid anhydrides including mixed anhydrides (e.g. acetic formic anhydride), or esters such as alkyl esters, orthoesters and (1-alkyl-2-pyridinyl) esters.

The reaction with an acid halide is preferably carried out in the presence of a base e.g. an inorganic base such as sodium hydroxide or an organic base such as triethylamine or pyridine. The reaction with an alkylchloroformate is preferably carried out in the presence of a base, e.g. potassium carbonate or triethylamine, in a solvent such as dimethylformamide. The reaction with an acid anhydride may be carried out in the absence or presence of a solvent such as pyridine.

In the reaction with an isocyanate or isothiocyanate compounds of formula (I) in which $R_{13}$ is other than hydrogen are conveniently prepared by carrying out the reaction in a solvent such as acetonitrile at an elevated temperature, e.g. reflux. Compounds of formula (I) in which $R_{13}$ is hydrogen may be prepared by heating with an appropriate organic isocyanate or isothiocyanate such as ethylcarbonisothiocyanatidate, at an elevated temperature followed by hydrolysis of the resulting ester, for example with a base such as aqueous ethanolic sodium hydroxide.

Amines of the formula (IV) where $R_{16}$ represents the group $R_1R_2NAlkQX(CH_2)_nY(CH_2)_m$- may be made by methods analogous to those described in German Offenlegungsschrifts 2,734,070, 2,821,410 and 2,821,409. For example, amines of formula (IV) in which X is oxygen or sulphur may be prepared by reacting a compound of formula (X)

$$R_1R_2NAlkQ—XH \qquad (X)$$

with the phthalimide derivative (XI)

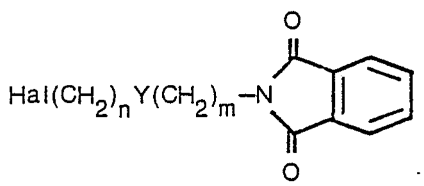

(XI)

where Hal is chlorine or bromine, in the presence of a base followed by removal of the phthalimide protecting group.

Amines of formula (IV) in which X is $—CH_2—$ may be prepared by reacting a compound of formula (XII) with a compound of formula (XIII)

$$R_1R_2NAlkQCH_2(CH_2)_nJ$$

(XII)

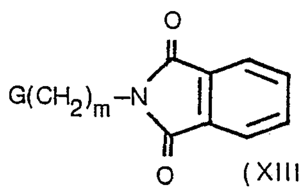

(XIII)

where either J is a leaving group e.g. halogen and G is a hydroxyl or thiol group, or J is a hydroxyl or thiol group and G is a leaving group e.g. halogen. The reaction is carried out in the presence of a base e.g. sodium hydride or potassium carbonate, in a solvent such as dimethylformamide or acetone respectively, to give an intermediate of formula (XIV)

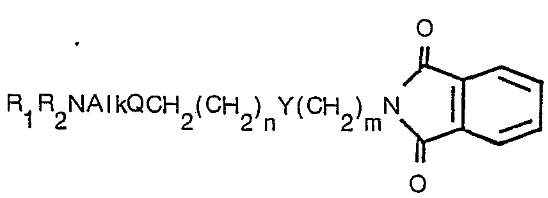

(XIV)

from which the amine (IV) in which X is $—CH_2—$ may be formed by cleavage of the phthalimide protecting group.

The intermediates of formulae (VII) and (VIII) may be prepared by methods analogous to those described in British Patent Specification No. 2023137A and European Patent Specification publication No. 0016565.

Where the product of any of the above processes is a free base and a salt is required, the salt may be formed in a conventional manner. Thus, for example, a generally convenient method of forming the

salts is to mix appropriate quantities of the free base and the acid in an appropriate solvent (s), e.g. an alcohol such as ethanol or an ester such as ethyl acetate.

The invention is illustrated by the following examples in which all temperatures are expressed in C°. In the following examples t.l.c. refers to thin layer chromatography carried out on silica using unless otherwise stated one of the following solvent systems;

A: ethyl acetate/water/isopropanol/0.88 ammonia (25:8:15:2)

B: methanol/0.88 ammonia (80.1).

Column chromatography was carried out on silica using methanol as eluent unless otherwise stated.

Preparation 1

6-[3-(1-Piperidinylmethyl)phenoxy]hexanamine

A suspension of 3-(1-piperidinylmethyl)phenol (3.9 g) and sodium hydride (0.49 g) in dry dimethylformamide (70 ml) was stirred at 25° for 6h. 2-(6-Bromohexyl)1H-isoindole-1,3-(2H)-dione (6.4 g) was added and the solution was stirred at 25° for 16h. The mixture was poured onto water and extracted with ethyl acetate. The organic extract was evaporated to leave a dark brown oil (8.6 g) which was used without further purification.

A solution of the oil and hydrazine hydrate (1.5 g) in ethanol (200 ml) was heated at reflux for 8h. Ether was added to the cooled solution and the precipitate was removed by filtration. The filtrate was distilled to give the *title compound* as a pale yellow oil (2.3 g) b.p. 170°/13.3 Pa (0.1 mm Hg).

The following compounds were similarly prepared from 3-(1-piperidinylmethyl)phenol [A] and the corresponding 2-(halosubstituted alkyl)-1H-isoindole-1,3-(2H)-dione.

(b) A (5.2 g) and 2-(5-Bromopentyl)-1H-isoindole-1,3-(2H)-dione (8 g) gave 5-[3-(1-piperidinyl-methyl)-phenoxy]pentanamine as a colourless oil (4.3 g) b.p. 150°/13.3 Pa (0.1 mm).

(c) A (2.7 g) and 2-[2-(2-Chloroethoxy)ethyl]-1H-isoindole-1,3-(2H)-dione (3.6 g) gave 2-[2-[3-(1-piperidinylmethyl)phenoxy]ethoxy]ethanamine as an oil (2 g), b.p. 180°/13.3 Pa (0.1 mm).

Preparation 2

2-[5-[3-Formylphenoxy]pentyl]-1H-isoindole-1,3-dione

3-Hydroxybenzaldehyde (6.48 g) and sodium hydride (80% 1.65 g) in dry dimethylformamide (120 ml) were stirred together at room temperature for 5h. N-(5-Bromopentyl)-phthalimide (14.3 g) was added and the mixture was stirred at 20° during 18h. The reaction mixture was poured onto water and extracted with ether. Evaporation of the ether gave the *title compound* (9.5 g) as a white solid.

T.l.c. silica; ethyl acetate Rf 0.7.

N.m.p. ($CDCl_3$) 0.0,s, (1H); 2.05—2.4,m, (4H); 2.5—2.7,m,(3H); 2.75—2.95,m,(1H); 6.0,t,(2H); 6.25,t,(2H); 7.8—8.7,m,(6H).

5-[3-(1,3-Dioxolan-2-yl)phenoxy]pentanamine

A solution of 2-[5-[3-formylphenoxy]pentyl]-1H-isoindole-1,3-dione (9.5 g), ethane-1,2-diol (2.4 g) and p-toluenesulphonic acid, monohydrate (75 mg) in benzene (150 ml) was heated under reflux using a Dean-Stark separator for 24h. The cooled solution was washed successively with sodium carbonate solution, water, sodium chloride solution, and evaporated *in vacuo*. The resulting oil was dissolved in tetrahydrofuran (150 ml) and stirred with hydrazine hydrate (4.9 g) at room temperature for 24h. The mixture was diluted with ether, filtered and the filtrate was distilled to give the *title compound* as a colourless oil (3.42 g) b.p. 185—189°/13.3 Pa (0.1 mm).

T.l.c. silica; methanol:ammonia 79:1 Rf 0.4

5-[[5-(3-Formylphenoxy)pentyl]amino]-1-methyl-1H-1,2,4-triazole-3-methanol

5-[3(1,3-Dioxolan-2-yl)phenoxy]pentanamine (3.26 g) and methyl N-[2-(acetyloxy)acetyl]-1-methyl-2-(phenylmethylene) hydrazinecarboximidothioate (3.98 g) were heated at 70° under water-pump vacuum for 3h. The resulting glass was dissolved in ethanol (25 ml) and stirred with 2N hydrochloric acid (100 ml) at room temperature for 18h. The solution was neutralised to pH 7 with potassium carbonate, and extracted with ethyl acetate to give a brown oil which was purified by column chromatography on silica using ethyl acetate followed by ethyl acetate:methanol (3:1) to give the *title compound* as a yellow solid.

T.l.c. silica; ethyl acetate:methanol 2:1 Rf 0.5.

N.m.r. ($CDCl_3$) 0.0,s,(1H); 2.5—3.0,m,(4H); 5.47,s,(2H); 5.8,t,(1H); 6.0,t,(2H); 6.55,s,(3H); 6.6,q,(2H); 6.6,s,(1H); 8—8.6,m,(6H).

Example 1

1-Methyl-$N^5$-[6-[3-(1-piperidinylmethyl)phenoxy]hexyl]-1H-1,2,4-triazole-3,5-diamine

6-[3-(1-Piperidinylmethyl)phenoxy]hexanamine (0.5 g) and methyl-N-cyano-1-methyl-2-(phenylmethylene)hydrazine carboximidothioate (0.4 g) were heated at 60° under water pump pressure for 4h. The resulting glass was dissolved in acetone, and the pH of the solution was adjusted

to pH1 with 2N hydrochloric acid. The solution was stirred at 25° for 1h, diluted with water and washed with ethyl acetate. The aqueous phase was basified with potassium carbonate and extracted with ethyl acetate. The organic extract was evaporated and the residual oil was triturated with cyclohexane to give the *title compound* as a white crystalline solid (0.41 g) m.p. 71—4°. T.l.c. system A, $R_f$ 0.5.

The following compounds were similarly prepared from the corresponding diamines and methyl-N-cyano-1-methyl-2-(phenylmethylene)hydrazine carboximidothioate [A].

(b) 5-[3-(1-Piperidinylmethyl)phenoxy]pentanamine (0.5 g) and A (0.42 g) gave 1-methyl-$N^5$-[5-[3-(1-piperidinylmethyl) phenoxy]pentyl]-1H-1,2,4-triazole-3,5-diamine as a pale yellow oil (0.35 g) purified by column chromatography. T.l.c. system A,$R_f$ 0.6. N.m.r. ($CDCl_3$) 2.8,t,(1H); 3.05—3.3,m,(3H); 6.04,s,(2H); 6.06,t,(2H); 6.54,s,(3H); 6.62,s,(2H); 6.6,q,(2H); 7.6,m,(4H) 8.0—8.7,m,(12H).

(c) 2-[2-[3-(1-Piperidinylmethyl)phenoxy]ethoxy]-ethanamine (1.0 g) and A (0.84 g) gave 1-methyl-$N^5$-[2-[2-[3-(1-piperidinylmethyl)phenoxy]ethoxy]ethyl]-1H-1,2,4-triazole-3,5-diamine (0.25 g) as a pale yellow oil, purified by column chromatography. T.l.c. system B, $R_f$ 0.58. N.m.r. ($CDCl_3$) 2.8,t,(1H); 3—3.3,m,(3H); 5.54,t,(1H); 5.8—6.6,m,(10H); 6.57,s,(2H); 6.7,s,(3H); 7.65,m,(4H); 8.5,m,(6H).

## Example 2

1-Methyl-5-[[5-[3-(1-piperidinylmethyl)phenoxy]pentyl]-amino]-111-1,2,4-triazole-3-methanol

5-[3-(1-Piperidinylmethyl)phenoxy]pentanamine (0.75 g) and methyl N-[(2-acetyloxy)acetyl]-1-methyl-2(phenylmethylene) hydrazinecarboximidothioate (0.84 g) were heated together, with stirring, at 80° for 2h under water pump vacuum to give an oil which was used without further purification. The oil was dissolved in toluene (15 ml) and, treated with 2N hydrochloric acid (5 ml). The resultant suspension was heated at 80—90° for 0.5h and stirred for 17h at 20°. The acidic layer was separated, washed with toluene and basified with potassium carbonate. The basic mixture was extracted with ethyl acetate and the organic extract was purified by column chromatography on silica using methanol, ethyl acetate (1:9) to give the *title compound* as a white solid (0.25 g) m.p. 82.5—83.5°.

| | | | |
|---|---|---|---|
| Assay found: | C, 65.0: | H, 8.2; | N, 17.5 |
| $C_{21}H_{33}N_5O_2$ requires: | C, 65.1; | H, 8.6; | N, 18.1% |

(b) In a similar manner, 2-[2-[3-(1-piperidinylmethyl)phenoxy]ethoxy]ethanamine (0.95 g) and methyl N-[(2-acetyloxy)acetyl]-1-methyl-2-(phenylmethylene)-hydrazinecarboximidothioate (0.94 g) gave 1-methyl-5-[[2-[2-[3-(1-piperidinylmethyl)phenoxy]ethoxy]ethyl]amino]-1H-1,2,4-triazole-3-methanol (0.3 g) as a pale yellow oil.

T.l.c. system A. $R_f$ 0.5

N.m.r. ($CDCl_3$) 2.76,t,(1H); 3.0—3.3,m,(3H); 5.30,t,(1H); 5.47,s,(2H); 5.90,m,(3H); 6.1—6.6,m,(11H); 7.60,m,(4H); 8.3—8.8,m,(6H).

## Example 3

N-[1-Methyl-5-[[5-[3-(piperidinylmethyl)phenoxy]pentyl]amino]-1H-1,2,4-triazole-3-yl]acetamide

Acetic anhydride (1 ml) was added to a solution of 1-methyl-$N^5$-[5-[3-(1-piperidinylmethyl)phenoxy]pentyl]-1H-1,2,4-triazole-3,5-diamine (0.7 g) in dry pyridine (15 ml) and the mixture was heated at 80° for 2h. Water was added followed by excess potassium carbonate and the mixture was stirred at 20° for 3h. The product was extracted into ethyl acetate and the combined organic extracts were evaporated to give the *title compound* as a yellow foam (0.4 g).

T.l.c. silica; ethyl acetate/ethanol/0.88 ammonia (20:3:2) $R_f$ 0.5.

N.m.r. ($CDCl_3$) 1.7,s,(1H); 2.8,t,(1H): 3—3.3,m,(3H); 5.8—6.07,m,(3H); 6.4—6.8,m,(7H); 7.5—8.0,m,(7H); 8.0—8.7,m,(12H).

## Example 4

3-Phenylmethyl-5-[[2-[2-[3-(1-piperidinylmethyl)phenoxy]ethoxy]ethyl]amino]-1H-1,2,4-triazole

*Dimethyldithio-N-phenacylcarbonimidinothioate*

A suspension of potassium carbonate (14.1 g) and dimethyl dithiocarbonimidinothioate hydroiodide (10.2 g) in acetone (150 ml) was stirred at 20° during 2h. Phenylacetyl chloride (6.8 g) was added dropwise and stirring was contained for 17h at 20°. The mixture was partially evaporated, diluted with water (300 ml) and extracted with ethyl acetate. The combined organic extracts were evaporated to give the *title compound* as a pale green oil (7.9 g) b.p. 160—164°, 7.998 Pa, (0.06 mm).

T.l.c. silica; ethyl acetate $R_f$ 0.8

3-Phenylmethyl-5-[[2-[2-[3-(1-piperidinylmethyl)phenoxy]ethoxy]ethyl]amino]-1H-1,2,4-triazole

A solution of 2-[2-[3-(1-piperidinylmethyl)phenoxy]ethoxy]ethanamine (1.5 g) in tetrahydrofuran (10 ml) was added dropwise to a solution of dimethyldithio-N-phenacylcarbonimidothioate (1.3 g) in tetrahydrofuran (20 ml) during 1h at 0° and the mixture was stirred at 20° for 16h. Hydrazine hydrate

(0.42 g) was added and the mixture was stirred at 20° for 17h, then heated under reflux for 3h. The mixture was partially evaporated, diluted with water and extracted with ethyl acetate. The organic extracts were evaporated to give an oil which was purified by column chromatography on silica using chloroformmethanol (9:1) to give the *title compound* as a white solid (0.3 g) m.p. 82.5—84.5°.

| | | | |
|---|---|---|---|
| Assay Found: | C, 66.8; | H, 7.4; | N, 15.3; |
| $C_{25}H_{33}N_5O_2.0.75H_2O$ requires: | C, 66.8; | H, 7.7; | N, 15.6% |

Example 5

N-Methyl-N[1]-[2-[2[3-(1-piperidinylmethyl)phenoxy]ethoxy]ethyl]-2-nitro-1,1-ethenediamine

A solution of 2-[2-[3-(1-piperidinylmethyl)phenoxy]ethoxy]ethanamine (0.4 g) and N-methyl-1-methylthio-2-nitroetheneamine (0.24 g) in water (20 ml) and ethanol (20 ml) was stirred at 50° for 5h. The solution was diluted with water (20 ml) acidified to pH 1 with dilute hydrochloric acid and washed with ethyl acetate (2 x 50 ml). The aqueous solution was basified to pH 9 with potassium carbonate and extracted with ethyl acetate (3 x 50 ml). Evaporation of these organic extracts gave a yellow oil which solidified when triturated with cyclohexane. The solid was recrystallised from ethyl acetate (10 ml) to give the *title compound* as a white powder (0.17 g) m.p. 125.5—126.5°

| | | | |
|---|---|---|---|
| Assay Found: | C, 60.28; | H, 7.98; | N, 14.64 |
| $C_{19}H_{30}N_4O_4$ requires: | C, 60.32; | H, 7.94; | N, 14.81% |

Example 6

N-[5-[3-(1-Piperidinylmethyl)phenoxy]pentyl]-N[1]methyl-2-nitro-1,1-ethenediamine

A solution of 5-[3-(1-piperidinylmethyl)-phenoxy]pentanamine (1.0 g) and 1-methylthio-1-methylamino-2-nitroethene (0.53 g) in water (20 ml) was stirred overnight at room temperature. The water was removed in vacuo and the resulting oil triturated with ethyl acetate to give a yellow solid which was dissolved in isopropanol:ethyl acetate (75:25). Addition of ether to the solution afforded the *title compound* (0.16 g) as a white crystalline solid, m.p. 112—113°.

| | | | |
|---|---|---|---|
| Found: | C, 61.3; | H, 8.3; | N, 14.0 |
| $C_{20}H_{32}N_4O_3.1H_2O$ requires: | C, 60.9; | H, 8.7; | N, 14.2%. |

Example 7

5-[[5-[3-[(2-Furanmethylamino)methyl]phenoxy]pentyl]amino]-1-methyl-1H-1,2,4-triazole-3-methanol

5-[[5-(3-Formylphenoxy)pentyl]amino]-1-methyl-1H-1,2,4-triazole-3-methanol (0.46 g) and 2-faranmethanamine (3.42 g) in ethanol (20 ml) were stirred together at 20° for 5h. Sodium borohydride (0.37 g) was added and the mixture stirred at 20° for 16h. The mixture was evaporated and the residue partitioned between 2N hydrochloric acid and ethyl acetate. The aqueous layer was washed with ethyl acetate, neutralised with potassium carbonate, and extracted with ethyl acetate. The combined extracts were dried and evaporated *in vacuo*, and the residual oil was chromatographed on silica gel using a mixture of methanol-ethyl acetate (1:1) to give the *title compound* as an oil (0.2 g).

T.l.c. silica:methanol Rf 0.5.

N.m.r. ($CDCl_3$) 1.6,m,(1H); 2.75,t,(1H); 3.0—3.3,m,(3H); 3.67.3.8,m,(2H); 5.4,s,(2H); 5.7,m,(1H); 6.2—6.8,m,(11H); 8.0—8.6,m,(6H).

Examples of Pharmaceutical Compositions

TABLETS

| | mg/tablet |
|---|---|
| Active ingredient | 100.00 |
| Microcrystalline Cellulose BPC | 198.50 |
| Magnesium stearate BP | 1.50 |
| Compression weight | 300.00 |

The active ingredient is sieved through a 250 $\mu$m sieve, blended with the excipients and compressed using 9.5 mm punches. Tablets of other strengths may be prepared by altering the compression weight and using punches to suit.

The tablets may be film coated with suitable film forming materials, e.g. methyl cellulose or hydroxypropyl methyl cellulose using standard techniques. Alternatively the tablets may be sugar coated.

Injection for Intravenous Administration

| | % w/v |
|---|---|
| Active ingredient | 1.00 |
| Water for injections B.P. to | 100.00 |

Sodium chloride may be added to adjust the tonicity of the solution and the pH may be adjusted to that of maximum stability and/or to facilitate solution of the active ingredient using either dilute acid or alkali.

The solution is prepared, clarified and filled into appropriate sized ampoules sealed by fusion of the glass. The injection is sterilised by heating in an autoclave using one of the acceptable cycles. Alternatively the solution may be sterilised by filtration and filled into sterile ampoules under aseptic conditions. The solution may be packed under an inert atmosphere of nitrogen.

Example 8

2-[3-[2-(N, N-Dimethylamino)methyl]phenyl]propoxy]ethyl]-N′-methyl-2-nitro-1,1-ethanediamine

3-[3-[2-Aminoethoxy]propyl]N,N-dimethyl-benzenemethanamine

A suspension of 3-[(N,N-dimethylamino)methyl]benzene propanol (2.5 g), potassium tertiary butoxide (0.56 g), 2-chloroethylamine hydrochloride (0.59 g) in diemthylformamide (10 ml) was stirred at 0—5° under a nitrogen atmosphere for 24h. The mixture was poured onto water (1 l) and extracted with ethyl acetate. The organic extract was evaporated to leave a yellow oil which was purified by column chromatography using methanol followed by methanol: 0.880 ammonia; (20:1) to give the *title compound* as a brown oil (73 mg).

T.l.c. system B Rf 0.2

2-[3-[2-(N,N-Dimethylamino)methyl]phenyl]propoxy]ethyl]-N′-methyl-2-nitro-1,1-ethanediamine

Following the method of Example 6, 3-[3-[2-aminoethoxy]propyl]N,N-dimethyl-benzenemethanamine (73 mg) gave the *title compound* as a white powder (38 mg).

T.l.c. system B Rf 0.35

N.m.r. ($CDCl_3$) -1 to 0, br.s.,(1H); 2.7—3.1,m,(4H); 3.1—3.5,br.s.,(1H); 3.41,s,(1H); 6.3—6.7,m,(6H); 6.62,s,(2H); 7.20,br.s.,(3H); 7.35,m,(2H); 7.80,s,(6H); 8.12,m,(2H).

The ability of the compounds of the invention to inhibit histamine induced gastric acid secretion has been demonstrated in the perfused rat stomach preparation referred to previously; the effectiveness of a compound conveniently being expressed in terms of its $ED_{50}$ (mg/kg) value. The results obtained for a representative number of compounds according to the invention are given below:

| | |
|---|---|
| 1(b) | 0.19 |
| 2(a) | 0.2 |
| 3 | 0.22 |
| 4 | 0.17 |

The compounds of the invention have in general low toxicity at doses at which they effectively inhibit gastric acid secretion. Thus for instance the compounds listed above produced no toxic effects when administered intravenously to anaesthetised rats at doses in excess of 4 times their $ED_{50}$ values.

## Claims

1. Compounds of the general formula (I)

$$R_1R_2N—Alk\text{-}Q—X(CH_2)_nY(CH_2)_mNH—B \quad (I)$$

and physiologically acceptable salts and hydrates thereof in which

B represents either the group

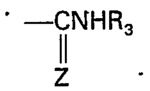 . or the group 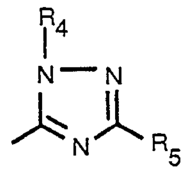 ;

$R_1$ represents $C_{3-8}$ cycloalkyl; aryl $C_{1-6}$ alkyl; trifluoro $C_{1-6}$ alkyl; heteroarylalkyl wherein the heteroaryl part is a monocyclic unsaturated ring containing 5 or 6 atoms selected from carbon, oxygen, nitrogen and sulphur, linked to the alkylene chain either through carbon or nitrogen and optionally substituted by $C_{1-3}$ alkyl, $C_{1-3}$ alkoxy, hydroxy, amino $C_{1-6}$ alkyl or halogen, and the alkyl part is a straight or branched alkylene chain with 1 to 4 carbon atoms; $C_{3-6}$ alkenyl or $C_{1-6}$ alkyl substituted by $C_{3-8}$ cycloalkyl or a $C_{1-16}$ straight or branched saturated alkylene chain optionally substituted by a hydroxy, amino, $C_{1-6}$ alkylamino, or di $C_{1-6}$ alkylamino group, or $R_1$ represents a $C_{1-16}$ straight branched saturated alkylene chain interrupted by an oxygen atom; with the proviso that when the alkylene chain is interrupted by an oxygen atom, then there must be at least two carbon atoms between that group and the nitrogen atom to which the group $R_1$ is attached;

$R_2$ represents hydrogen or a $C_{1-4}$ alkyl group;

or $R_1$ and $R_2$ together with the nitrogen atom to which they are attached form a 5—10 membered ring which may be saturated or may contain at least one double bond, may be unsubstituted or may be substituted by one or more $C_{1-3}$ alkyl groups or a hydroxy group and/or may contain another heteroatom selected from oxygen or sulphur;

Alk represents a straight or branched alkylene chain of 1 to 6 carbon atoms;

Q represents a furan of thiophen ring in which incorporation into the rest of the molecule is through bonds at the 2- and 5- positions, the furan ring optionally bearing a further substituent $R_7$ adjacent to the group $R_1R_2$—N—Alk-, or Q represents a benzene ring in which incorporation into the rest of the molecule is through bonds at the 1- and 3- or 1- and 4- positions;

$R_7$ represents halogen, or $C_{1-4}$ alkyl which may be substituted by hydroxy or $C_{1-4}$ alkoxy;

X and Y, which may be the same or different, each represent oxygen or sulphur, or one of X and Y represents —$CH_2$— and the other represents oxygen or sulphur;

n and m, which may be the same or different, each represent 2 or 3;

$R_3$ represents hydrogen, $C_{1-6}$ alkyl, $C_{3-6}$ alkenyl, or $C_{1-6}$ alkoxy $C_{1-6}$ alkyl;

Z represents $CHNO_2$ or $NR_8$ where $R_8$ is nitro, cyano, $C_{1-6}$ alkylsulphonyl or arylsulphonyl;

$R_4$ represents hydrogen, $C_{1-6}$ alkyl, $C_{3-6}$ alkenyl, aryl $C_{1-6}$ alkyl, or $C_{2-6}$ alkyl substituted by hydroxy or $C_{1-6}$ alkoxy; and

$R_5$ represents hydrogen, $C_{1-6}$ alkyl, $C_{3-6}$ alkenyl, aryl $C_{1-6}$ alkyl, hydroxy $C_{1-6}$ alkyl, acyloxy $C_{1-6}$ alkyl, (wherein the acyl portion is aroyl, aryl $C_{2-7}$ alkanoyl or $C_{1-6}$ alkanoyl), $C_{1-6}$ alkoxy $C_{1-6}$ alkyl, aryloxy $C_{1-6}$ alkyl, aryl $C_{1-6}$ alkyloxy $C_{1-6}$ alkyl, amino $C_{1-6}$ alkyl, $C_{1-6}$ alkylamino $C_{1-6}$ alkyl, di $C_{1-6}$ alkylamino $C_{1-6}$ alkyl, hydroxy or $C_{1-6}$ alkoxy or the group $NR_9R_{10}$ where $R_9$ represents hydrogen or $C_{1-6}$ alkyl;

$R_{10}$ represents hydrogen, $C_{1-6}$ alkyl, $C_{1-6}$ alkyl substituted by hydroxy or $C_{1-3}$ alkoxy, $C_{3-6}$ alkenyl, aryl $C_{1-6}$ alkyl or heteroaryl $C_{1-6}$ alkyl, or $R_{10}$ may represent the group $COR_{11}$ where $R_{11}$ represents hydrogen, $C_{1-6}$ alkyl, aryl, aryl $C_{1-6}$ alkyl, $C_{1-6}$ alkoxy, heteroaryl or heteroaryl $C_{1-6}$ alkyl or $R_{10}$ represents the group $SO_2R_{12}$ where $R_{12}$ represents $C_{1-6}$ alkyl or aryl, or $R_{10}$ represents the group

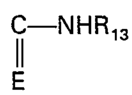

where E is oxygen or sulphur and

$R_{13}$ represents hydrogen, $C_{1-6}$ alkyl, $C_{3-8}$ cycloalkyl, aryl or aryl $C_{1-6}$ alkyl,

or $R_9$ and $R_{10}$ taken together represent the group $=CR_{14}R_{15}$ where $R_{14}$ represents aryl or heteroaryl and

$R_{15}$ represents hydrogen or $C_{1-6}$ alkyl; and wherein

aryl as a group or part of a group means phenyl or phenyl substituted with one or more $C_{1-3}$ alkyl or $C_{1-3}$ alkoxy groups or halogen atoms;

heteroaryl as a group or part of a group within the definition of the group $R_5$ means a 5 or 6 membered monocyclic unsaturated ring which may contain one or more heteroatoms selected from oxygen, nitrogen or sulphur.

2. Compounds as claimed in Claim 1, in which the groups $R_1$, $R_2$ and $R_7$ have the following meanings:

$R_1$: alkyl containing up to 16 carbon atoms, $C_{5-7}$ cycloalkyl, $C_{3-6}$ alkenyl, aryl $C_{1-6}$ alkyl, $C_{1-4}$ alkyl substituted by a trifluoromethyl group, hydroxy $C_{2-4}$ alkyl, $C_{1-3}$ alkoxy, $C_{2-4}$ alkyl, or di-$C_{1-3}$ alkylamino

$C_{1-6}$ alkyl, or heteroarylalkyl where the heterocyclic portion represents a furyl, thienyl, pyrrolyl, pyridinyl, pyrimidinyl, triazinyl, oxazolyl, triazolyl, or thiazolyl ring and the alkylene portion is methylene, ethylene or propylene;

$R_2$: hydrogen or $C_{1-4}$ alkyl; or

$R_1R_2N$ may represent a 5—8 membered ring optionally containing one double bond and/or substituted by one or two $C_{1-3}$ alkyl groups or a hydroxy group and/or containing an oxygen or sulphur atom;

$R_7$: bromine atom or a $C_{1-3}$ alkyl group or a $C_{1-3}$ alkoxy $C_{1-3}$ alkyl group and

B represents the group

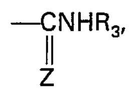

in which

$R_3$ and Z have the following meanings:

$R_3$: hydrogen, $C_{1-4}$ alkyl or $C_{1-3}$ alkoxy $C_{2-4}$ alkyl;

Z: $CHNO_2$ or $NR_8$ where $R_8$ is nitro, cyano or $C_{1-4}$ alkylsulphonyl; or

B represents the group

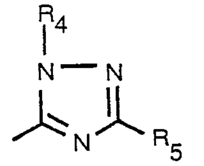
;

in which $R_4$ and $R_5$ have the following meanings:

$R_4$: hydrogen, $C_{1-4}$ alkyl or hydroxy $C_{2-4}$ alkyl;

$R_5$: hydrogen, hydroxy, $C_{1-4}$ alkyl, hydroxy $C_{1-4}$ alkyl, $C_{1-3}$ alkoxy $C_{1-4}$ alkyl, phenyl $C_{1-3}$ alkyl, $C_{2-4}$ alkanoyloxy $C_{1-4}$ alkyl, amino $C_{1-4}$ alkyl, amino, $C_{1-4}$ alkylamino, di-$C_{1-4}$ alkylamino, phenyl $C_{1-3}$ alkylamino, or a heteroaryl $C_{1-3}$ alkylamino group where the heteroaryl ring is 5 or 6 membered and contains one heteroatom; or the group $NHCOR_{11}$ where $R_{11}$ represents hydrogen, $C_{1-3}$ alkyl, $C_{1-3}$ alkoxy, furyl, pyridyl, thiazolyl, thienyl, or phenyl optionally substituted by a $C_{1-3}$ alkyl or $C_{1-3}$ alkoxy group; or the group $NHSO_2R_{12}$ where $R_{12}$ represents $C_{1-3}$ alkyl, or phenyl optionally substituted by a $C_{1-3}$ alkyl or $C_{1-3}$ alkoxy group; or the group $—NHCONHR_{13}$ where $R_{13}$ is $C_{1-3}$ alkyl, $C_{5-7}$ cycloalkyl, or phenyl optionally substituted by a $C_{1-3}$ alkyl or $C_{1-3}$ alkoxy group; or the group $N=CHR_{14}$ where $R_{14}$ is a phenyl or pyridyl group; and the group Alk is the group $(CH_2)_p$ where p is 1, 2 or 3.

3. Compounds as claimed in Claim 1 or 2 in which the group Q is a benzene ring in which incorporation into the rest of the molecule is through bonds at the 1- and 3-positions; one of X and Y is oxygen and the other is oxygen or methylene; and n and m are both two.

4. Compounds as claimed in Claim 1 corresponding to formula (III)

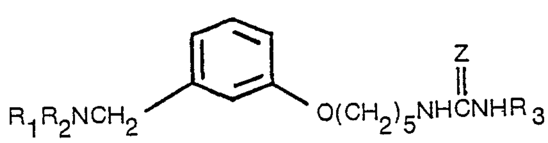
(III)

in which $R_1R_2N$ forms a pyrrolidino, piperidino, hexamethylenimino, tetrahydropyridino or 4-methylpiperidino group; Z represents $CHNO_2$ or $NR_8$ where $R_8$ is methylsulphonyl; and $R_3$ represents hydrogen, $C_{1-4}$ alkyl or $C_{1-3}$ alkoxy $C_{2-4}$ alkyl.

5. Compounds as claimed in Claim 1 corresponding to formula (II)

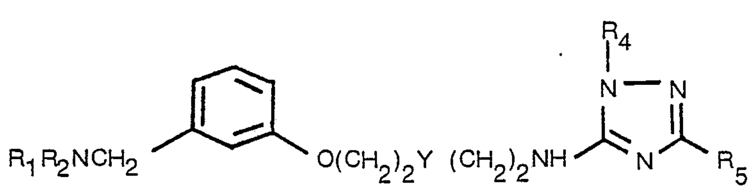
(II)

where $R_1$ and $R_2$ are methyl groups or together with the nitrogen atom to which they are attached form a pyrrolidino, piperidino, hexamethylenimino, tetrahydropyridino or 4-methylpiperidino group; $R_4$ is hydrogen or methyl; and $R_5$ is an amino, hydroxy $C_{1-6}$ alkyl, $C_{2-7}$ alkanoylamino, $C_{2-7}$ alkanoyloxy $C_{1-6}$ alkyl, $C_{1-6}$ alkoxy $C_{1-6}$ alkyl, amino $C_{1-6}$ alkyl, benzyl, formamido, $C_{1-6}$ alkoxycarbonylamino, hydroxy, aroylamino or phenylcarbamoylamino group; and Y represents oxygen or methylene.

6. Compounds as claimed in Claim 5 in which $R_5$ is amino, $C_{2-7}$ alkanoylamino, hydroxy $C_{1-6}$ alkyl or benzyl.

7. A compound as claimed in Claim 1, which is N-[5-[3-(1-piperidinylmethyl)phenoxy]pentyl]-N'-methyl-2-nitro-1,1-ethenediamine or a physiologically acceptable salt thereof.

8. Compounds as claimed in Claim 1 in which B represents

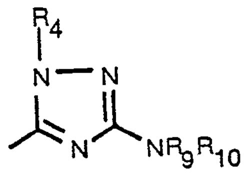

$R_1$ represents $C_{3-8}$ cycloalkyl, aryl $C_{1-6}$ alkyl, $C_{3-6}$ alkenyl, trifluoro $C_{1-6}$ alkyl, heteroaryl $C_{1-4}$ alkyl or $C_{1-6}$ alkyl substituted by $C_{3-8}$ cycloalkyl, amino, $C_{1-6}$ alkylamino or di $C_{1-6}$ alkylamino; or $R_1$ represents a $C_{1-16}$ straight or branched saturated alkylene chain which may be optionally substituted by a hydroxy group; or $R_1$ represents a $C_{1-16}$ straight or branched saturated alkylene chain interrupted by an oxygen atom, with the proviso that when the alkylene chain is interrupted by an oxygen atom, then there must be at least two carbon atoms between that group and the nitrogen atom to which the group $R_1$ is attached;

$R_2$ represents hydrogen or a $C_{1-4}$ alkyl group;

or $R_1$ and $R_2$ together with the nitrogen atom to which they are attached form a 5—10 membered ring which may be saturated or may contain at least one double bond, may be unsubstituted or may be substituted by one or more $C_{1-3}$ alkyl groups or a hydroxy group and/or may contain another heteroatom; selected from oxygen and sulphur; and

$R_9$ represents hydrogen or $C_{1-6}$ alkyl;

$R_{10}$ represents hydrogen, $C_{1-6}$ alkyl, $C_{1-6}$ alkyl substituted by hydroxy or $C_{1-3}$ alkoxy, $C_{3-6}$ alkenyl, aryl $C_{1-6}$ alkyl or heteroaryl $C_{1-6}$ alkyl

or $R_9$ and $R_{10}$ together represent the group $=CR_{14}R_{15}$ where $R_{14}$ represents aryl or heteroaryl and $R_{15}$ represents hydrogen or $C_{1-6}$ alkyl;

wherein aryl and heteroaryl (within the definitions of $R_1$ and $R_5$) are as defined in claim 1.

9. Compounds as claimed in Claim 1 in which B is

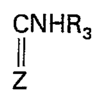

where Z is $CHNO_2$.

10. A process for the production of compounds of formula (I) as defined in Claim 1, characterised in that

a) for the production of compounds in which B represents the group

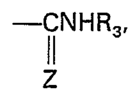

a primary amine of formula $R_{16}NH_2$ (IV) is reacted with a compound of general formula (V)

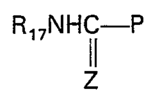 (V)

in which one of the groups $R_{16}$ and $R_{17}$ represents the group $R_1R_2NAlkQX(CH_2)_nY(CH_2)_m$- and the other represents the group $R_3$, and P is a leaving group.

b) for the production of compounds in which B represents the group.

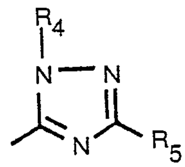 ;

where $R_5$ is other than an alkoxy or acyloxyalkyl group, or the group $—N=CR_{14}R_{15}$, a compound of formula (VII)

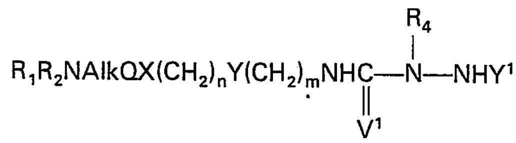 (VII)

in which $V^1$ is

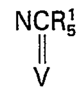

or NCN and $Y^1$ is hydrogen, where V is oxygen or sulphur and $R_5^1$ is a group as defined for $R_5$ or is a group convertible thereto under the conditions of the reaction or represents halogen or alkoxy, or $V^1$ is NH and $Y^1$ is

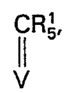

is cyclised;

c) for the production of compounds in which Alk is $CH_2$ and B represents the group

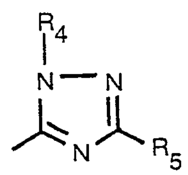 ;

in which $R_5$ is other than the group $—N=CR_{14}R_{15}$, an intermediate formed by reacting a compound of formula (IX)

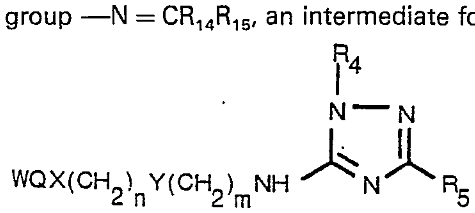 (IX)

in which W represents the group —CHO, with a reagent $R_1R_2NH$, is reduced:

d) for the production of compounds in which B represents the group

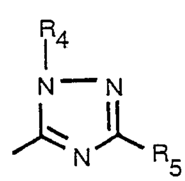 ;

in which $R_5$ is other than the group $—N=CR_{14}—R_{15}$, a compound of formula (I) in which B represents the group

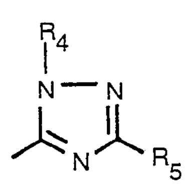 ;

in which $R_5$ is other than the group $—N=CR_{14}R_{15}$ and in which $R_1$ is hydrogen is reacted with an aldehyde $R_1^1$-CHO under reducing conditions, the group $R_1^1$ having a meaning such that the resultant group $R_1^1CH_2$ represents a group $R_1$ as defined in formula (I);

e) for the production of compounds in which B represents the group

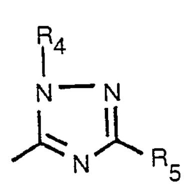 ;

in which $R_5$ is an acyloxyalkyl group, the corresponding hydroxyalkyl compound is treated with an appropriate acid at elevated temperature;

f) for the production of compounds in which B represents the group

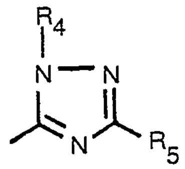 ;

in which the group $R_5$ represents the group $—N = CR_{14}R_{15}$, a compound of formula (I) in which $R_5$ is an $NH_2$ group is reacted with an aldehyde or ketone $R_{14}R_{15}CO$;

g) for the production of compounds in which B represents the group

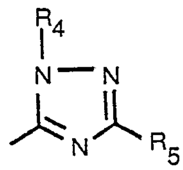 ;

in which $R_5$ is the group $NR_9R_{10}$ where $R_{10}$ is $—COR_{11}$, $—SO_2R_{12}$ or $—C(=E)NHR_{13}$, a compound of formula (I) in which $R_5$ is the group $—NHR_9$ and $R_1$, $R_2$, $R_4$ and $R_9$ are as defined in formula (I) or are groups readily convertible thereto is treated with a reagent capable of replacing the hydrogen atom in the group $NHR_9$ by the group $R_{10}$; and where the compound of formula (I) is produced in the form of a free base, optionally converting the free base into a salt.

11. A pharmaceutical composition comprising a compound as claimed in any of Claims 1 to 9 together with at least one pharmaceutically acceptable carrier or diluent, and optionally one or more further active ingredients.

**Patentansprüche**

1. Verbindungen der allgemeinen Formel (I):

$$R_1R_2N—Alk\text{-}Q—X(CH_2)_nY(CH_2)_mNH—B \qquad (I)$$

und die physiologisch annehmbaren Salze und Hydrate davon, worin B entweder für die Gruppe

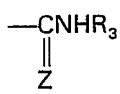

oder die Gruppe

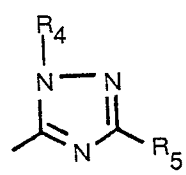

steht, $R_1$ für $C_{3-8}$-Cycloalkyl, Aryl-$C_{1-6}$-alkyl, Trifluor-$C_{1-6}$-alkyl, Heteroarylalkyl, wobei der Heteroarylteil ein monocylischer ungesättigter Ring, enthaltend 5 oder 6 Atome, ausgewählt aus Kohlenstoff, Sauerstoff, Stickstoff und Schwefel, ist, der mit der Alkylenkette entweder durch Kohlenstoff oder Stickstoff verbunden ist, und gegebenenfalls durch $C_{1-3}$-Alkyl, $C_{1-3}$-Alkoxy, Hydroxy, Amino-$C_{1-6}$-alkyl oder Halogen substituiert ist, und der Alkylteil eine gerade oder verzweigte Alkylenkette mit 1 bis 4 Kohlenstoffatomen ist, $C_{3-6}$-Alkenyl oder $C_{1-6}$-Alkyl, substituiert durch $C_{3-8}$-Cycloalkyl oder eine gerade oder verzweigte gesättigte $C_{1-16}$-Alkylenkette, gegebenfalls substituiert durch eine Hydroxy-, Amino-, $C_{1-6}$-alkylamino-oder Di-$C_{1-6}$-alkylaminogruppe, steht oder $R_1$ für eine gerade oder verzweigte gesättigte $C_{1-16}$-Alkylenkette, die durch ein Sauerstoffatom unterbrochen ist, steht, mit der Maßgabe, daß, wenn die Alkylenkette durch ein Sauerstoffatom unterbrochen ist, mindestens zwei Kohlenstoffatome zwischen dieser Gruppe und dem Stickstoffatom, an das die Gruppe $R_1$ angefügt ist, sein müssen, $R_2$ für Wasserstoff oder eine $C_{1-4}$-Alkylgruppe steht, oder $R_1$ und $R_2$ zusammen mit dem Stickstoffatom, an das sie angefügt sind, einen 5-bis 10-gliedrigen Ring bilden, der gesättigt sein kann oder mindestens eine Doppelbindung enthalten kann, der unsubstituiert sein kann oder durch eine oder mehrere $C_{1-3}$-Alkyl-

gruppen oder eine Hydroxygruppe substituiert sein kann und/oder ein weiteres Heteroatom, ausgewählt aus Sauerstoff oder Schwefel, enthalten kann, Alk für eine gerade oder verzweigte Alkylenkette mit 1 bis 6 Kohlenstoffatomen steht, Q für einen Furan- oder Thiophenring steht, dessen Einarbeitung in den Rest des Moleküls durch Bindungen in 2- und 5-Stellungen erfolgt ist, wobei die Furanring gegebenenfalls einen weiteren Substituenten $R_7$, angrenzend an die Gruppe $R_1R_2$-N—Alk- trägt, oder Q für einen Benzolring steht, dessen Einarbeitung in den Rest des Moleküls durch Bindungen in 1- und 3- oder 1- und 4-Stellungen erfolgt ist, $R_7$ für Halogen oder $C_{1-4}$-Alkyl, das durch Hydroxy oder $C_{1-4}$-Alkoxy substituiert sein kann, steht, X und Y, die gleich oder verschieden sein kann, jeweils für Sauerstoff oder Schwefel stehen oder eine Gruppe von X und Y für —$CH_2$— steht und die andere Gruppe für Sauerstoff oder Schwefel steht, n und m, die gleich oder verschieden sein können, jeweils den Wert 2 oder 3 haben, $R_3$ für Wasserstoff, $C_{1-6}$-Alkyl, $C_{3-6}$-Alkenyl, oder $C_{1-6}$-Alkoxy-$C_{1-6}$-alkyl steht, Z für $CHNO_2$ oder $NR_8$ steht, wobei $R_8$ für Nitro, Cyano, $C_{1-6}$-Alkylsulfonyl oder Arylsulfonyl steht, $R_4$ für Wasserstoff, $C_{1-6}$-Alkyl, $C_{3-6}$-Alkenyl, Aryl-$C_{1-6}$-alkyl oder $C_{2-6}$-Alkyl, das durch Hydroxy oder $C_{1-6}$-Alkoxy substituiert ist, steht und $R_5$ für Wasserstoff, $C_{1-6}$-Alkyl, $C_{3-6}$-Alkenyl, Aryl-$C_{1-6}$-alkyl, Hydroxy-$C_{1-6}$-alkyl, Acyloxy-$C_{1-6}$-alkyl (wobei dier Acylteil Aroyl, Aryl-$C_{2-7}$-alkanoyl oder $C_{1-6}$-Alkanoyl ist), $C_{1-6}$-Alkoxy-$C_{1-6}$-alkyl, Aryloxy-$C_{1-6}$-alkyl, Aryl-$C_{1-6}$alkyloxy-$C_{1-6}$-alkyl, Amino-$C_{1-6}$alkyl, $C_{1-6}$-Alkylamino-$C_{1-6}$-alkyl, Di-$C_{1-6}$-alkylamino-$C_{1-6}$-alkyl, Hydroxy oder $C_{1-6}$-Alkoxy oder die Gruppe $NR_9R_{10}$ steht, worin $R_9$ für Wasserstoff oder $C_{1-6}$-Alkyl steht, $R_{10}$ für Wasserstoff, $C_{1-6}$-Alkyl, $C_{1-6}$-Alkyl, substituiert durch Hydroxy oder $C_{1-3}$-Alkoxy, $C_{3-6}$-Alkenyl, Aryl-$C_{1-6}$-alkyl oder Heteroaryl-$C_{1-6}$-alkyl steht oder $R_{10}$ die Gruppe $COR_{11}$ bedueten kann, worin $R_{11}$ für Wasserstoff, $C_{1-6}$-Alkyl, Aryl, Aryl-$C_{1-6}$-alkyl, $C_{1-6}$-Alkoxy, Heteroaryl oder Heteroaryl-$C_{1-6}$-alkyl steht, oder $R_{10}$ die Gruppe $SO_2R_{12}$ darstellt, worin $R_{12}$ für $C_{1-6}$-Alkyl oder Aryl steht, oder $R_{10}$ für die Gruppe

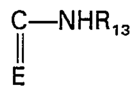

steht, wobei E für Sauerstoff oder Schwefel steht und $R_{13}$ für Wasserstoff, $C_{1-6}$-Alkyl, $C_{3-8}$-Cycloalkyl, Aryl oder Aryl-$C_{1-6}$-alkyl steht, oder $R_9$ und $R_{10}$ miteinander die Gruppe $=CR_{14}R_{15}$ bilden, wobei $R_{14}$ für Aryl oder Heteroaryl steht und $R_{15}$ für Wasserstoff oder $C_{1-6}$-Alkyl steht, und wobei Aryl als Gruppe oder Teil einer Gruppe die Bedeutung Phenyl oder Phenyl, substituiert mit einer oder mehreren $C_{1-3}$-Alkyl- oder $C_{1-3}$-Alkoxygruppen oder Halogenatomen, hat, Heteroaryl als Gruppe oder Teil einer Gruppe innerhalb der Definition der Gruppe $R_5$ einen 5- oder 6-gliedrigen monocyclischen ungesättigten Ring bedeutet, der ein oder mehrere Heteroatome, ausgewählt aus Sauerstoff, Stickstoff oder Schwefel, enthalten kann.

2. Verbindung nach Anspruch 1, dadurch gekennzeichnet, daß die Gruppen $R_1$, $R_2$ und $R_7$ die folgenden Bedeutungen haben:

$R_1$: Alkyl mit bis zu 16 Kohlenstoffatomen, $C_{5-7}$-Cycloalkyl, $C_{3-6}$-Alkenyl, Aryl-$C_{1-6}$-alkyl, $C_{1-4}$-Alkyl, substituiert durch eine Trifluormethylgruppe, Hydroxy-$C_{2-4}$-alkyl, $C_{1-3}$-Alkoxy-$C_{2-4}$-alkyl oder Di-$C_{1-3}$-alkylamino-$C_{1-6}$-alkyl oder Heteroarylalkyl, wobei der heterocyclische Teil einen Furyl-, Thienyl-, Pyrrolyl-, Pyridinyl-, Pyrimidinyl-, Triazinyl-, Oxazolyl-, Triazolyl- oder Thiazolylring darstellt und der Alkylenteil Methylen, Ethylen oder Propylen ist,

$R_2$: Wasserstoff oder $C_{1-4}$-Alkyl oder

$R_1R_2N$ einen 5- bis 8-gliedrigen Ring darstellen können, der gegebenenfalls eine Doppelbindung enthält und/oder durch eine oder zwei $C_{1-3}$-Alkylgruppen oder eine Hydroxygruppe substituiert ist und/oder ein Sauerstoffoder Schwefelatom enthält,

$R_7$: Bromatom oder eine $C_{1-3}$-Alkylgruppe oder eine $C_{1-3}$-Alkoxy-$C_{1-3}$-alkylgruppe und

B: die Gruppe

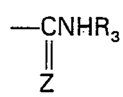

bedeutet, wobei $R_3$ und Z die folgenden Bedeutungen haben:

$R_3$: Wasserstoff, $C_{1-4}$-Alkyl oder $C_{1-3}$-Alkoxy-$C_{2-4}$-alkyl,

Z: $CHNO_2$ oder $NR_8$, wobei $R_8$ für Nitro, Cyano oder $C_{1-4}$-Alkylsulfonyl steht, oder

B: die Gruppe:

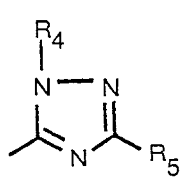 ;

darstellt, worin $R_4$ und $R_5$ die folgenden Bedeutungen haben:

$R_4$: Wasserstoff, $C_{1-4}$-Alkyl oder Hydroxy-$C_{2-4}$-alkyl,

$R_5$: Wasserstoff, Hydroxy, $C_{1-4}$-Alkyl, Hydroxy-$C_{1-4}$-alkyl, $C_{1-3}$-Alkoxy-$C_{1-4}$-alkyl, Phenyl-$C_{1-3}$-alkyl, $C_{2-4}$-Alkanoyloxy-$C_{1-4}$-alkyl, Amino-$C_{1-4}$-alkyl, Amino, $C_{1-4}$-Alkylamino, Di-$C_{1-4}$-alkylamino, Phenyl-$C_{1-3}$-alkylamino oder eine Heteroaryl-$C_{1-3}$-alkylaminogruppe, wobei der Heteroarylring 5- oder 6-gliedrig ist und ein Heteroatom enthält, oder die Gruppe $NHCOR_{11}$, wobei $R_{11}$ für Wasserstoff, $C_{1-3}$-Alkyl, $C_{1-3}$-Alkoxy, Furyl, Pyridyl, Thiazolyl, Thienyl oder Phenyl, gegebenenfalls substituiert durch eine $C_{1-3}$-Alkyl- oder $C_{1-3}$-Alkoxygruppe, steht oder die Gruppe $NHSO_2R_{12}$, wobei $R_{12}$ für $C_{1-3}$-Alkyl oder Phenyl, gegebenenfalls substituiert durch eine $C_{1-3}$-Alkyl- oder $C_{1-3}$-Alkoxygruppe, steht oder die Gruppe —$NHCONHR_{15}$, wobei $R_{15}$ für $C_{1-3}$-Alkyl, $C_{5-7}$-Cycloalkyl oder Phenyl, gegebenenfalls substituiert durch eine $C_{1-3}$-Alkyl- oder $C_{1-3}$-Alkoxygruppe, steht, oder die Gruppe $N = CHR_{14}$, wobei $R_{14}$ eine Phenyl- oder Pyridylgruppe ist, und die Gruppe Alk die Gruppe $(CH_2)_p$ ist, wobei p den Wert 1, 2 oder 3 hat.

3. Verbindungen nach Anspruch 1 oder 2, dadurch gekennzeichnet, daß die Gruppe Q eine Benzolring ist, dessen Einarbeitung in den Rest des Moleküls durch Bindungen in 1- und 3-Stellungen erfolgt ist, daß eine der Gruppierungen X und Y Sauerstoff ist und daß die andere Sauerstoff oder Methylen ist, und daß n und m beide den Wert 2 haben.

4. Verbindungen nach Anspruch 1 entsprechend der Formel (III):

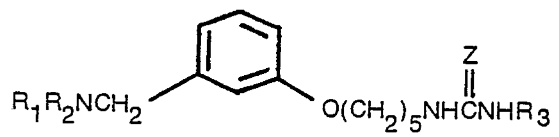

(III)

worin $R_1R_2N$ eine Pyrrolidino-, Piperidino-, Hexamethylenimino-, Tetrahydropyridino- oder 4-Methylpiperidinogruppe bildet, Z für $CHNO_2$ oder $NR_8$ steht, wobei $R_8$ für Methylsulfonyl steht, und $R_3$ für Wasserstoff, $C_{1-4}$-Alkyl oder $C_{1-3}$-Alkoxy-$C_{2-4}$-alkyl steht.

5. Verbindungen nach Anspruch 1 entsprechend der Formel (II):

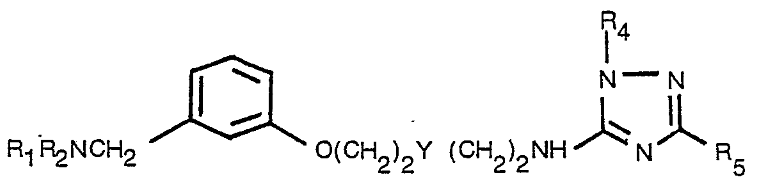

(II)

worin $R_1$ und $R_2$ Methylgruppen sind oder zusammen mit dem Stickstoffatom, an das sie angefügt sind, eine Pyrrolidino-, Piperidino-, Hexamethylenimino-, Tetrahydropyridino- oder 4-Methylpiperidinogruppe bilden, $R_4$ für Wasserstoff oder Methyl steht und $R_5$ eine Amino-, Hydroxy-$C_{1-6}$-alkyl-, $C_{2-7}$-Alkanoylamino-, $C_{2-7}$-Alkanoyloxy-$C_{1-6}$-alkyl-, $C_{1-6}$-Alkoxy-$C_{1-6}$-alkyl, Amino-$C_{1-6}$-alkyl, Benzyl-, Formamido-, $C_{1-6}$-Alkoxycarbonylamino-, Hydroxy-, Aroylamino- oder Phenylcarbamoylaminogruppe ist und Y für Sauerstoff oder Methylen steht.

6. Verbindungen nach Anspruch 5, dadurch gekennzeichnet, daß $R_5$ für Amino, $C_{2-7}$-Alkanoylamino, Hydroxy-$C_{1-6}$-alkyl oder Benzyl steht.

7. Verbindung nach Anspruch 1, nämlich N-[5-[3-(1-Piperidinylmethyl)-phenoxy]-pentyl]-N'-methyl-2-nitro-1,1-ethendiamin oder ein physiologisch annehmbares Salz davon.

8. Verbindungen nach Anspruch 1, dadurch gekennzeichnet, daß B

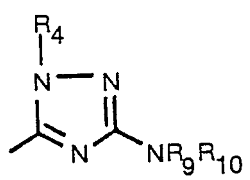

darstellt, $R_1$ für $C_{3-8}$-Cycloalkyl, Aryl-$C_{1-6}$-alkyl, $C_{3-6}$ Alkenyl, Trifluor-$C_{1-6}$-alkyl, Heteroaryl-$C_{1-4}$-alkyl oder $C_{1-6}$-Alkyl, substituiert durch $C_{3-8}$-Cycloalkyl, Amino, $C_{1-6}$-Alkylamino oder Di-$C_{1-6}$-alkylamino, steht oder $R_1$ für eine gerade oder verzweigte gesättigte $C_{1-16}$-Alkylenkette steht, die gegebenenfalls durch eine Hydroxygruppe substituiert sein kann, oder $R_1$ eine gerade oder verzweigte gesättigte $C_{1-16}$-Alkylenkette bedeutet, die durch ein Sauerstoffatom unterbrochen ist, mit der Maßgabe, daß, wenn die Alkylenkette durch ein Sauerstoffatom unterbrochen ist, dann mindestens zwei Kohlenstoffatome zwischen dieser Gruppe und dem Stickstoffatom, an das die Gruppe $R_1$ angefügt ist, sein müssen, $R_2$ für Wasserstoff oder eine $C_{1-4}$-Alkylgruppe steht, oder $R_1$ und $R_2$ zusammen mit dem Stickstoffatom, an das sie angefügt sind, einen 5- bis 10-gliedrigen Ring bilden, der gesättigt sein kann oder der mindestens eine Doppelbindung enthalten kann, der unsubstituiert sein kann oder der durch eine oder mehrere $C_{1-3}$-Alkylgruppen oder eine Hydroxygruppe substituiert sein kann, und/oder der ein weiteres

Heteroatom, ausgewählt aus Sauerstoff und Schwefel, enthalten kann, und $R_9$ für Wasserstoff oder $C_{1-6}$-Alkyl steht, $R_{10}$ für Wasserstoff, $C_{1-6}$-Alkyl, $C_{1-6}$-Alkyl, substituiert durch Hydroxy oder $C_{1-3}$-Alkoxy, $C_{3-6}$-Alkenyl, Aryl-$C_{1-6}$-alkyl oder Heteroaryl-$C_{1-6}$-alkyl steht, oder $R_9$ und $R_{10}$ zusammen die Gruppe $=CR_{14}R_{15}$ darstellen, wobei $R_{14}$ für Aryl oder Heteroaryl steht und $R_{15}$ für Wasserstoff oder $C_{1-6}$-Alkyl steht, wobei Aryl und Heteroaryl (innerhalb der Definitionen von $R_1$ und $R_5$) wie im Anspruch 1 definiert sind.

9. Verbindungen nach Anspruch 1, dadurch gekennzeichnet, daß B für

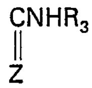

steht, wobei Z die Bedeutung $CHNO_2$ hat.

10. Verfahren zur Herstellung von Verbindungen der Formel (I) nach Anspruch 1, dadurch gekennzeichnet, daß man

a) zur Herstellung von Verbindungen, bei denen B die Gruppe

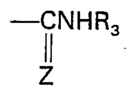

bedeutet, ein primäres Amin der Formel

$$R_{16}NH_2 \qquad \text{(IV)}$$

mit einer Verbindung der allgemeinen Formel (V):

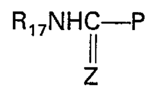

(V)

wobei eine der Gruppen $R_{16}$ und $R_{17}$ die Gruppe $R_1R_2NAlkQX(CH_2)_nY(CH_2)_m$- und die andere die Gruppe $R_3$ darstellt und P eine Austrittsgruppe ist, umsetzt,

b) zur Herstellung von Verbindungen, bei denen B die Gruppe

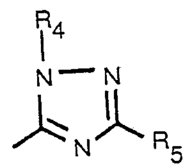

worin $R_5$ eine andere Bedeutung als eine Alkoxy- oder Acyloxyalkylgruppe hat, oder die Gruppe $—N=CR_{14}R_{15}$ eine Verbindung der Formel (VII):

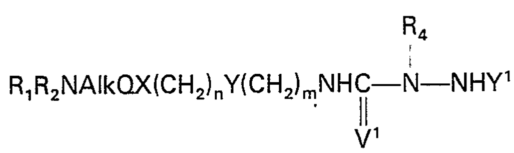

(VII)

worin $V^1$ für

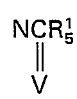

oder NCN steht und $Y^1$ für Wasserstoff steht, wobei V Sauerstoff oder Schwefel ist und $R_5^1$ eine Gruppe wie für $R_5$ definiert oder eine in eine solche Gruppe bei den Reaktionsbedingungen umwandelbare Gruppe ist oder Halogen oder Alkoxy darstellt, oder $V^1$ für NH steht und $Y^1$ für

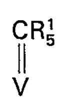

steht, cyclisiert,

c) zur Herstellung von Verbindungen, bei denen Alk für $CH_2$ steht und B die Gruppe

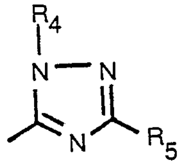

darstellt, wobei $R_5$ eine andere Bedeutung als die Gruppe —N = $CR_{14}R_{15}$ hat, ein Zwischenprodukt, gebildet durch Umsetzung einer Verbindung der Formel (IX):

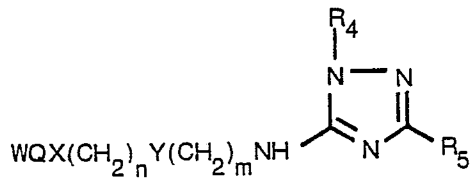

wobei W für die Gruppe —CHO steht, mit einem Reagenz $R_1R_2NH$ reduziert,

d) zur Herstellung von Verbindungen, bei denen B die Gruppe

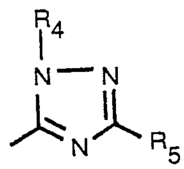

darstellt, wobei $R_5$ eine andere Bedeutung als die Gruppe —N = $CR_{14}R_{15}$ hat, eine Verbindung der Formel (I), worin B die Gruppe

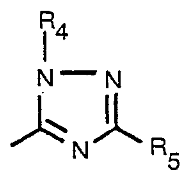

darstellt, worin $R_5$ eine andere Bedeutung als die Gruppe —N = $CR_{14}R_{15}$ hat, und worin $R_1$ für Wasserstoff steht, mit einem Aldehyd $R_1^1$—CHO unter reduzierenden Bedingungen umsetzt, wobei die Gruppe $R_1^1$ eine derartige Bedeutung hat, daß die resultierende Gruppe $R_1^1CH_2$ eine Gruppe $R_1$, wie im Zusammenhang mit der Formel (I) definiert, bedeutet,

e) zur Herstellung von Verbindungen, bei denen B die Gruppe

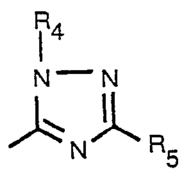

darstellt, wobei $R_5$ eine Acyloxyalkylgruppe ist, die entsprechende Hydroxyalkylverbindung mit einer geeigneten Säure bei erhöhter Temperatur behandelt,

f) zur Herstellung von Verbindungen, bei denen B die Gruppe

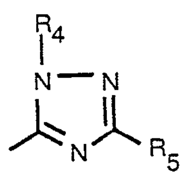

darstellt, wobei die Gruppe $R_5$ für die Gruppe —N = $CR_{14}R_{15}$ steht, eine Verbindung der Formel (I), bei der $R_5$ eine $NH_2$-Gruppe ist, mit einem Aldehyd oder Keton $R_{14}R_{15}CO$ umsetzt,

g) zur Herstellung von Verbindungen, bei denen B die Gruppe

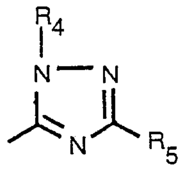

darstellt, wobei $R_5$ die Gruppe $NR_9R_{10}$ ist, wobei $R_{10}$ für $-COR_{11}$, $-SO_2R_{12}$ oder $-C(=E)NHR_{13}$ steht, eine Verbindung der Formel (I), bei der $R_5$ die Gruppe $-NHR_9$ ist und $R_1$, $R_2$, $R_4$ und $R_9$ wie im Zusammenhang mit der Formel (I) definiert sind oder Gruppen sind, die ohne weiteres in solche Gruppen umwandelbar sind, mit einem Reagenz behandelt, das dazu imstande ist, das Wasserstoffatom in der Gruppe $NHR_9$ durch die Gruppe $R_{10}$ auszutauschen, und wenn die Verbindung der Formel (I) in Form einer freien Base gebildet wird, man die freie Base gegebenenfalls in ein Salz umwandelt.

11. Arzneimittel, dadurch gekennzeichnet, daß es eine Verbindung nach einem der Ansprüche 1 bis 9 zusammen mit mindestens einem pharmazeutisch annehmbaren Träger oder Verdünnungsmittel und gegebenenfalls einem oder mehreren weiteren Wirkstoffen enthält.

## Revendications

1. Composés de formule générale (I)

$$R_1R_2N—Alk—Q—X(CH_2)_nY(CH_2)_mNH—B \qquad (I)$$

et leurs sels et hydrates physiologiquement accetables dans laquelle
B représente ou le groupe

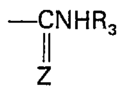 ou le groupe 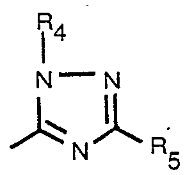 ;

$R_1$ représente un cycloalkyle en $C_3$—$C_8$; un aryl-(alkyle en $C_1$—$C_6$); un trifluoro-(alkyle en $C_1$—$C_6$); un hétéroarylalkyle dans lequel la partie hétéroaryle est un monocycle insaturé, contenant 5 ou 6 atomes choisis parmi le carbone, l'oxygène, l'azote et le soufre, lié à la chaîne alkylène par l'intermédiaire ou du carbone ou de l'azote et éventuellement substitué par un alkyle en $C_1$—$C_3$, un alcoxy en $C_1$—$C_3$, un hydroxy, un amino-(alkyle en $C_1$—$C_6$) ou un halogène, et la partie alkyle est une chaîne alkylène droite ou ramifiée ayant de 1 à 4 atomes de carbone; un alcényle en $C_3$—$C_6$ ou un alkyle en $C_1$—$C_6$ substitué par un cycloalkyle en $C_3$—$C_8$ ou une chaîne alkylène saturée droite ou ramifiée en $C_1$—$C_{16}$ éventuellement substituée par un groupe hydroxy, amino, (alkyle en $C_1$—$C_6$)-amino ou di-(alkyle en $C_1$—$C_6$)-amino, ou $R_1$ représente une chaîne alkylène saturée droite ou ramifiée en $C_1$—$C_{16}$ interrompue par un atome d'oxygène; à condition que, lorsque la chaîne alkylène est interrompue par un atome d'oxygène, il doive se trouver au moins deux atomes de carbone entre ce groupe et l'atome d'azote auquel est fixé le groupe $R_1$;

$R_2$ représente l'hydrogène ou un groupe alkyle en $C_1$—$C_4$;

ou $R_1$ et $R_2$ forment, avec l'atome d'azote auquel ils sont fixés, un cycle de 5 à 10 chaînons qui peut être saturé on peut contenir au moins une double liaison, peut être non substitué ou peut être substitué par un ou plusieurs groupes alkyle en $C_1$—$C_3$ ou par un groupe hydroxy et/ou peut contenir un autre hétéroatome choisi entre l'oxygène ou le soufre;

Alk représente une chaîne alkylène droite ou ramifiée de 1 à 6 atomes de carbone;

Q représente un cycle furanne ou thiophène relié au reste de la molécule par des liaisons en positions 2 et 5, le cycle furanne portant éventuellement un autre substituant $R_7$ adjacent au groupe $R_1R_2$—N—Alk-, ou Q représente un noyau benzénique relié au reste de la molécule par des liaisons en positions 1 et 3 ou 1 et 4;

$R_7$ représente un halogène ou un alkyle en $C_1$—$C_4$ qui peut être substitué par un hydroxy ou un alcoxy en $C_1$—$C_4$;

X et Y, qui peuvent être identiques ou différents, représentent chacun l'oxygène ou le soufre, ou l'un parmi X et Y représente $—CH_2—$ et l'autre représente l'oxygène ou le soufre;

n et m, qui peuvent être identiques ou différents, représentent chacun 2 ou 3;

$R_3$ représente l'hydrogène, un alkyle en $C_1$—$C_6$, un alcényle en $C_3$—$C_6$ ou un (alcoxy en $C_1$—$C_6$)-(alkyle en $C_1$—$C_6$);

Z représente $CHNO_2$ ou $NR_8$ dans lequel $R_8$ est le nitro, le cyano, un (alkyle en $C_1$—$C_6$)-sulfonyle ou un arylsulfonyle;

$R_4$ représente l'hydrogène, un alkyle en $C_1$—$C_6$, un alcényle en $C_3$—$C_6$, un aryl-(alkyle en $C_1$—$C_6$) ou un alkyle en $C_2$—$C_6$ substitué par un hydroxy ou un alcoxy en $C_1$—$C_6$; et

$R_5$ représente l'hydrogène, un alkyle en $C_1$—$C_6$, un alcényle en $C_3$—$C_6$, un aryl-(alkyle en $C_1$—$C_6$), un hydroxy-(alkyle en $C_1$—$C_6$), un acyloxy-(alkyle en $C_1$—$C_6$) (dans lequel la portion acyle est un aroyle, un aryl-(alcanoyle en $C_2$—$C_7$) ou un alcanoyle en $C_1$—$C_6$), un (alcoxy en $C_1$—$C_6$)-(alkyle en $C_1$—$C_6$), un aryloxy-(alkyle en $C_1$—$C_6$), un aryl-(alkyloxy en $C_1$—$C_6$)-(alkyle en $C_1$—$C_6$), un amino-(alkyle en $C_1$—$C_6$), un (alkylamino en $C_1$—$C_6$)-(alkyle en $C_1$—$C_6$), un di-(alkylamino en $C_1$—$C_6$)-(alkyle en $C_1$—$C_6$), un hydroxy ou un alcoxy en $C_1$—$C_6$ ou le groupe $NR_9R_{10}$ dans lequel $R_9$ représente l'hydrogène ou un alkyle en $C_1$—$C_6$;

$R_{10}$ représente l'hydrogène, un alkyle en $C_1$—$C_6$, un alkyle en $C_1$—$C_6$ substitué par l'hydroxy ou un alcoxy en $C_1$—$C_3$, un alcényle en $C_3C_6$, un aryl-(alkyle en $C_1$—$C_6$) ou un hétéroaryl-(alkyle en $C_1$—$C_6$), ou $R_{10}$ peut représenter le groupe $COR_{11}$ dans lequel $R_{11}$ représente l'hydrogène, un alkyle en $C_1$—$C_6$, un aryle, un aryl-(alkyle en $C_1$—$C_6$), un alcoxy en $C_1$—$C_6$, un hétéroaryle ou un hétéroaryl-(alkyle en $C_1$—$C_6$) ou $R_{10}$ représente le groupe $SO_2R_{12}$ dans lequel $R_{12}$ représente un alkyle en $C_1$—$C_6$ ou un aryle, ou $R_{10}$ représente le groupe

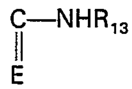

dans lequel E est l'oxygène ou le soufre et

$R_{13}$ représente l'hydrogène, un alkyle en $C_1$—$C_6$, un cycloalkyle en $C_3$—$C_8$, un aryle ou un aryl-(alkyle en $C_1$—$C_6$);

ou $R_9$ et $R_{10}$ représentent ensemble le groupe $=CR_{14}R_{15}$ dans lequel $R_{14}$ représente un aryle ou un hétéroaryle et

$R_{15}$ représente l'hydrogène ou un alkyle en $C_1$—$C_6$; et dans lequel

aryle en tant que groupe ou que partie de groupe signifie le phényle ou le phényle substitué par un ou plusieurs groupes alkyle en $C_1$—$C_3$ ou alcoxy en $C_1$—$C_3$ ou des atomes d'halogène;

hétéroaryle en tant que groupe ou que partie de groupe dans la définition du groupe $R_5$ signifie un monocycle insaturé à 5 ou 6 chaînons qui peut contenir un ou plusieurs hétéroatomes choisis parmi l'oxygène, l'azote ou le soufre.

2. Composés selon la revendication 1, caractérisés en ce que les groupes $R_1$, $R_2$ et $R_7$ ont les significations suivantes:

$R_1$: alkyle contenant jusqu'à 16 atomes de carbone, cycloalkyle en $C_5$—$C_7$, alcényle en $C_3$—$C_6$, aryl-(alkyle en $C_1$—$C_6$), alkyle en $C_1$—$C_4$ substitué par un groupe trifluorométhyle, hydroxy-(alkyle en $C_2$—$C_4$), (alcoxy en $C_1$—$C_3$)-(alkyle en $C_2$—$C_4$) ou di-(alkyl en $C_1$—$C_3$)-amino-(alkyle en $C_1$—$C_6$) ou hétéroarylalkyle dans lequel la portion hétérocyclique représente un cycle furyle, thiényle, pyrrolyle, pyridinyle, pyrimidinyle, triazinyle, oxazolyle, triazolyle ou thiazolyle et la portion alkylène est le méthylène, l'éthylène ou le propylène;

$R_2$: hydrogène ou alkyle en $C_1$—$C_4$; ou

$R_1R_2N$ peut représenter un cycle de 5 à 8 chaînons contenant éventuellement une double liaison et/ou substitué par un ou deux groupes alkyle en $C_1$—$C_3$ ou un groupe hydroxy et/ou contenant un atome d'oxygène ou de soufre;

$R_7$: atome de brome ou groupe alkyle en $C_1$—$C_3$ ou groupe (alcoxy en $C_1$—$C_3$)-(alkyle en $C_1$—$C_3$) et

B représente le groupe

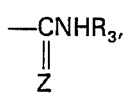

dand lequel $R_3$ et Z ont les significations suivantes:

$R_3$: hydrogène alkyle en $C_1$—$C_4$ ou (alcoxy en $C_1$—$C_3$)-(alkyle en $C_2$—$C_4$);

Z: $CHNO_2$ ou $NR_8$ dans lequel $R_8$ est un nitro, cyano ou (alkyl en $C_1$—$C_4$)-sulfonyle; ou

B représente le groupe

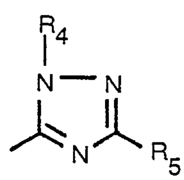

dans lequel $R_4$ et $R_5$ ont les significations suivantes:

$R_4$: hydrogène, alkyle en $C_1$—$C_4$ ou hydroxy-(alkyle en $C_2$—$C_4$);

$R_5$: hydrogène, hydroxy, alkyle en $C_1$—$C_4$, hydroxy-(alkyle en $C_1$—$C_4$), (alcoxy en $C_1$—$C_3$)-(alkyle

en $C_1$—$C_4$), phényl-(alkyle en $C_1$—$C_3$), (alcanoyloxy en $C_2$—$C_4$)-(alkyle en $C_1$—$C_4$), amino-(alkyle en $C_1$—$C_4$), amino, (alkyl en $C_1$—$C_4$)-amino, di-(alkyl en $C_1$—$C_4$)-amino, phényl-(alkyl en $C_1$—$C_3$)-amino ou un groupe hétéroaryl-(alkyl en $C_1$—$C_3$) amino dans lequel le cycle hétéroaryle comporte 5 ou 6 chaînons et contient un hétéroatome; ou le groupe $NHCOR_{11}$ dans lequel $R_{11}$ représente l'hydrogène, un groupe alkyle en $C_1$—$C_3$, alcoxy en $C_1$—$C_3$, furyle, pyridyle, thiazolyle, thiényle, ou phényle éventuellement substitué par un groupe alkyle en $C_1$—$C_3$ ou alcoxy en $C_1$—$C_3$; ou le groupe $NHSO_2R_{12}$ dans lequel $R_{12}$ représente un groupe alkyle en $C_1$—$C_3$, ou phényle éventuellement substitué par un groupe alkyle en $C_1$—$C_3$ ou alcoxy en $C_1$—$C_3$; ou le groupe —$NHCONHR_{13}$ dans lequel $R_{13}$ est un alkyle en $C_1$—$C_3$, cycloalkyle en $C_5$—$C_7$, ou phényle éventuellement substitué par un groupe alkyle en $C_1$—$C_3$ ou alcoxy en $C_1$—$C_3$; ou le groupe $N = CHR_{14}$ dans lequel $R_{14}$ est un groupe phényle ou pyridyle; et le groupe Alk est le groupe $(CH_2)_p$ dans lequel p est égal à 1, 2 ou 3.

3. Composés selon la revendication 1 ou 2, caractérisés en ce que le groupe Q est un noyau benzénique relié au reste de la molécule par des liaisons en positions 1 et 3; l'un parmi X et Y est l'oxygène et l'autre est l'oxygène ou le méthylène; et n et m sont chacun égaux à 2.

4. Composés selon la revendication 1 correspondant à la formule (III)

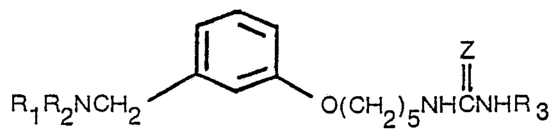

(III)

dans laquelle $R_1R_2N$ forme un groupe pyrrolidino, pipéridino, hexaméthylène-imino, tétrahydropyridino ou méthyl-4 pipéridino; Z représente $CHNO_2$ ou $NR_8$ dans lequel $R_8$ est le méthylsulfonyle; et $R_3$ représente l'hydrogène, un alkyle en $C_1$—$C_4$ ou un (alcoxy en $C_1$—$C_3$)-(alkyle en $C_2$—$C_4$).

5. Composés selon la revendication 1 correspondant à la formule (II)

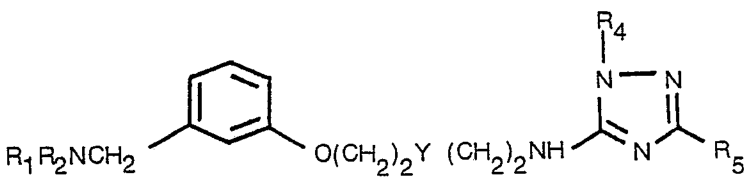

(II)

dans laquelle $R_1$ et $R_2$ sont des groupes méthyle ou forment, avec l'atome d'azote auquel ils sont fixés, un groupe pyrrolidino, pipéridino, hexaméthylène-imino, tétrahydropyridino ou méthyl-4 pipéridino; $R_4$ est l'hydrogène ou le méthyle; et $R_5$ est un groupe amino, hydroxy-(alkyle en $C_1$—$C_6$), (alcanoyl en $C_2$—$C_7$)-amino, (alcanoyloxy en $C_2$—$C_7$)-(alkyle en $C_1$—$C_6$), (alcoxy en $C_1$—$C_6$)-(alkyle en $C_1$—$C_6$), amino-(alkyle en $C_1$—$C_6$), benzyle, formamido, (alcoxy en $C_1$—$C_6$)-carbonylamino, hydroxy, aroylamino ou phénylcarbamoylamino; et Y représente l'oxygène ou le méthylène.

6. Composés selon la revendication 5, caractérisés en ce que $R_5$ est l'amino, un (alcanoyl en $C_2$—$C_7$)-amino, un hydroxy-(alkyle en $C_1$—$C_6$) ou le benzyle.

7. Composé selon la revendication 1, caractérisé en ce qu'il est la N-[[(pypéridinyl-1 méthyl)-3 phénoxyl]-5 pentyl]-N[1]-méthyl-nitro-2 éthènediamine-1,1 ou un de ses sels physiologiquement acceptables.

8. Composés selon la revendication 1, caractérisés en ce que B représente

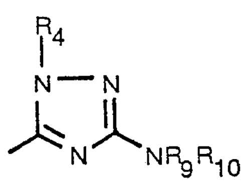

$R_1$ représente un cycloalkyle en $C_3$—$C_8$, un aryl-(alkyle en $C_1$—$C_6$), un alcényle en $C_3$—$C_6$, un trifluoro-(alkyle en $C_1$—$C_6$), un hétéroaryl-(alkyle en $C_1$—$C_4$) ou un alkyle en $C_1$—$C_6$ substitué par un cyclalkyle en $C_3$—$C_8$, un amino, un (alkyl en $C_1$—$C_6$)-amino ou un di-(alkyl en $C_1$—$C_6$)-amino; ou $R_1$ représente une chaîne alkylène saturée droite ou ramifiée en $C_1$—$C_{16}$ qui peut être éventuellement substitueé par un groupe hydroxy; ou $R_1$ représente une chaîne alkylène saturée droite ou ramifiée en $C_1$—$C_{16}$ interrompue par un atome d'oxygène, à condition que, lorsque la chaîne alkylène est interrompue par un atome d'oxygène, il doive se trouver au moins deux atomes de carbone entre ce groupe et l'atome d'azote auquel est fixé le groupe $R_1$;

$R_2$ représente l'hydrogène ou un groupe alkyle en $C_1$—$C_4$;

ou $R_1$ et $R_2$ forment, avec l'atome d'azote auquel ils sont fixés, un cycle de 5 à 10 chaînons qui peut être saturé ou peut contenir au moins une double liaison, peut être non substitué ou peut être substitué par un ou plusieurs groupes alkyle en $C_1$—$C_3$ ou par un groupe hydroxy et/ou peut contenir un autre hétéroatome choisi entre l'oxygène ou le soufre; et

$R_9$ représente l'hydrogène ou un alkyle en $C_1$—$C_6$;

$R_{10}$ représente l'hydrogène, un alkyle en $C_1$—$C_6$, un alkyle en $C_1$—$C_6$ substitué par un hydroxy ou un alcoxy en $C_1$—$C_3$, un alcényle en $C_3$—$C_6$, un aryl-(alkyle en $C_1$—$C_6$) ou un hétéroaryl-(alkyle en $C_1$—$C_6$)

ou $R_9$ et $R_{10}$ représentent ensemble le groupe $=CR_{14}R_{15}$ dans lequel $R_{14}$ représente un aryle ou un hétéroaryle et $R_{15}$ représente l'hydrogène ou un alkyle en $C_1$—$C_6$;

dans lesquelles aryle et hétéroaryle (dans les définitions de $R_1$ et $R_5$) sont définis dans la revendication 1.

9. Composés selon la revendication 1, caractérisés en ce que B est

$CNHR_3$
‖
Z

dans lequel Z est $CHNO_2$.

10. Procédé pour la production des composés de formule (I) selon la revendication 1, caractérisé en ce que

a) pour la production des composés dans lesquels B représente le groupe

—$CNHR_3$
‖
Z

on fait réagir une amine primaire de formule $R_{16}NH_2$ (IV) avec un composé de formule générale (V)

$R_{17}NHC$—P (V)
‖
Z

dans laquelle l'un des groupes $R_{16}$ et $R_{17}$ représente le groupe $R_1R_2NAlkQX(CH_2)_nY(CH_2)_m$- et l'autre représente le groupe $R_3$, et P est un groupe partant;

b) pour la production des composés dans lesquels B représente le groupe

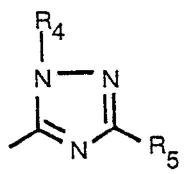

dans lequel $R_5$ est autre qu'un groupe alcoxy ou acyloxyalkyle, ou le groupe $—N=CR_{14}R_{15}$, on cyclise un composé de formule (VII)

$R_4$
$R_1R_2NAlkQX(CH_2)_nY(CH_2)_mNHC$—N—$NHY^1$ (VII)
‖
$V^1$

dans laquelle $V^1$ est

$NCR^1_5$
‖
V

ou NCN et $Y^1$ est l'hydrogène, où V est l'oxygène ou le soufre et $R^1_5$ est un groupe tel que défini pour $R_5$ ou est un groupe pouvant être transformé en $R_5$ dans les conditions de la réaction ou représente un halogène ou un alcoxy, ou $V^1$ est NH et $Y^1$ est

$CR^1_5$;
‖
V

c) pour la production des composés dans lesquels Alk est $CH_2$ et B représente le groupe

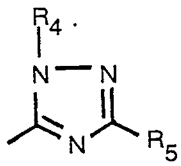

dans lequel $R_5$ est autre que le groupe $—N=CR_{14}R_{15}$, on réduit un intermédiaire formé par la réaction d'un composé de formule (IX)

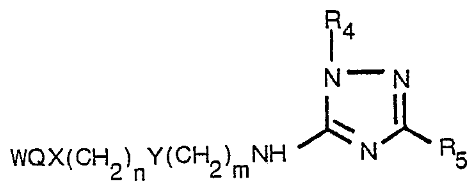

dans laquelle W représente le groupe —CHO, avec un réactif $R_1R_2NH$;

d) pour la production des composés dans lesquels B représente le groupe

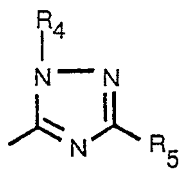

dans lequel $R_5$ est autre que le groupe $—N=CR_{14}—R_{15}$, on fait réagir un composé de formule (I) dans laquelle B représente le groupe

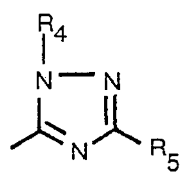

dans laquelle $R_5$ est autre que le groupe $—N=CR_{14}R_{15}$ et dans laquelle $R_1$ est l'hydrogène, avec un aldéhyde $R_1^1$—CHO dans des conditions réductrices, le groupe $R_1^1$ ayant une signification telle que le groupe résultant $R_1^1CH_2$ représente un groupe $R_1$ tel que défini dans la formule (I);

e) pour la production des composés dans lesquels B représente le groupe

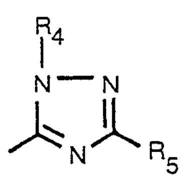

dans lequel $R_5$ est un groupe acyloxyalkyle, on traite le composé hydroxyalkyle correspondant avec un acide approprié à température élevée;

f) pour la production des composés dans lesquels B représente le groupe

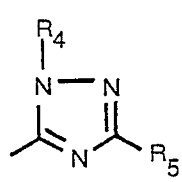

dans lequel le groupe $R_5$ représente le groupe $—N=CR_{14}R_{15}$, on fait réagir un composé de formule (I), dans lequel $R_5$ est un groupe $NH_2$, avec un aldéhyde ou une cétone $R_{14}R_{15}CO$;

g) pour la production des composés dans lesquels B représente le groupe

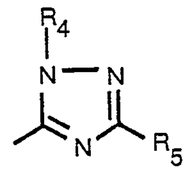

dans lequel $R_5$ est le groupe $NR_9R_{10}$ où $R_{10}$ est $—COR_{11}$, $—SO_2R_{12}$ ou $—C(=E)NHR_{13}$, on traite un composé de formule (I), dans laquelle $R_5$ est le groupe $—NHR_9$ et $R_1$, $R_2$, $R_4$ et $R_9$ sont tels que définis dans la formule (I) ou sont des groupes facilement transformables en ces derniers, avec un réactif capable de remplacer l'atome d'hydrogène du groupe $NHR_9$ par le groupe $R_{10}$; et où le composé de formule (I) est produit sous forme d'une base libre, en transformant éventuellement la base libre en un sel.

11. Composition pharmaceutique comprenant un composé selon l'une quelconque des revendications 1 à 9 ainsi qu'au moins un véhicule ou diluant pharmaceutiquement acceptable et éventuellement un ou plusierus autres principes actifs.